# EUROPEAN PATENT SPECIFICATION

(11) **EP 2 998 307 B1**
(45) Date of publication and mention of the grant of the patent: **31.10.2018**
(21) Application number: 14798157.5
(22) Date of filing: 20.03.2014
(51) Int. Cl.: C07F 9/40, C07H 19/10, C07H 19/207, A61K 31/661, A61P 31/14

(54) **PHOSPHORIC ACID/PHOSPHONIC ACID DERIVATIVES AND MEDICINAL USES THEREOF**
PHOSPHORSÄURE-/PHOSPHONSÄUREDERIVATE UND DEREN MEDIZINISCHE VERWENDUNGEN
DÉRIVÉS D'ACIDE PHOSPHORIQUE/PHOSPHONIQUE ET LEURS UTILISATIONS EN MÉDECINE

(30) Priority: 14.05.2013 CN 201310176630
(43) Date of publication of application: 23.03.2016
(73) Proprietor: Beijing SL Pharmaceutical Co., Ltd., Haidian District Beijing 100049 (CN)
(72) Inventor: WANG, Jianming, Beijing 100070 (CN); CHEN, Xiaoguang, Beijing 100070 (CN); CHEN, Junping, Beijing 100070 (CN); FAN, Shan, Beijing 100070 (CN)
(74) Representative: CMS Cameron McKenna Nabarro Olswang LLP
(86) International application number: PCT/CN2014/000302
(87) International publication number: WO 2014/183462

(56) References cited:
- WO-A1-2012/142075
- CN-A- 1 045 395
- TW-A- 201 311 715
- DATABASE CA [Online] CHEMICAL ABSTRACTS SERVICE, COLUMBUS, OHIO, US; OKAMOTO, YOSHIKI ET AL: "Intramolecular rearrangement of bis(3,4-methylenedioxyphenyl) methylphosphonate induced by single electron-transfer oxidation", XP002765558, retrieved from STN Database accession no. 1997:363839
- DATABASE CA [Online] CHEMICAL ABSTRACTS SERVICE, COLUMBUS, OHIO, US; KAWAI, TSUTOMU ET AL: "Preparation of 2'-deoxy-5-fluorouridine derivatives as antitumor agents", XP002765561, retrieved from STN Database accession no. 1991:632776
- DATABASE CA [Online] CHEMICAL ABSTRACTS SERVICE, COLUMBUS, OHIO, US; KAWAI, TSUTOMU ET AL: "2'-Deoxy-5-fluorouridine derivatives as neoplasm inhibitors.", XP002765559, retrieved from STN Database accession no. 1990:119354
- DATABASE CA [Online] CHEMICAL ABSTRACTS SERVICE, COLUMBUS, OHIO, US; DUTRA, GERARD A.: "O-Aryl, N-phosphonomethylglycinonitriles", XP002765560, retrieved from STN Database accession no. 1978:509957

## Description

### Technical Field

The present invention relates to novel liver-targeted prodrug derivatives of pharmacologically active molecules containing a phosphate or phosphonate group therein and nontoxic, pharmaceutically acceptable salts, hydrates or solvates thereof, and pharmaceutical compositions containing the prodrug derivatives and nontoxic, pharmaceutically acceptable salts, hydrates or solvates thereof as active ingredients and suitable excipients, and use of the prodrug derivatives and nontoxic, pharmaceutically acceptable salts, hydrates or solvates thereof, and the pharmaceutical compositions containing the prodrug derivatives and nontoxic, pharmaceutically acceptable salt, hydrates or solvates thereof as active ingredients for the manufacture of a medicament for treating hepatopathy or metabolic diseases.

### Background of the Related Art

Liver is the target organ of viral hepatitis, liver cirrhosis and liver cancer, and also is the major organ of glucolipid metabolism. Study on therapeutic techonologies, therapeutic methods and therapeutic drugs of these diseases has made significant progress in recent ten years, but it is still far from meeting clinical requirements. For example, for the chronic viral hepatitis, the efficacy of interferon is unstable, and its side-effect is greater; and lamivudine, an anti-HBV drug, is prone to generate drug resistance, and adefovir dipivoxil has dose-limiting nephrotoxicity; and the long-term use of ribavirin, the only small-molecule anti-HCV drug, can cause severe hematotoxicity. For liver cancer, the conventional chemotherapy drug has great systematic side-effect. For liver fibrosis and liver cirrhosis, there are no clinically safe and effective drugs. For diabetes and hyperlipidemia, clinically, new more efficient therapeutic methods are also urgently needed.

Nucleoside phosphoric acid/phosphonic acid is an important part of an antiviral drug. Nucleoside analogues, such as lamivudine, telbivudine, entecavir, ribavirin, PSI-6130 and the like, all form nucleotide analogues with pharmacological activity by phosphorylation to exert antiviral effect.

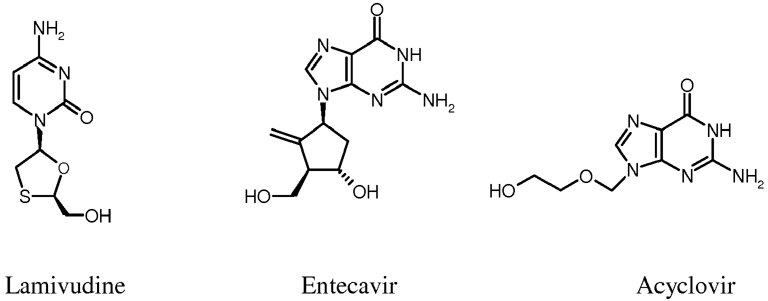

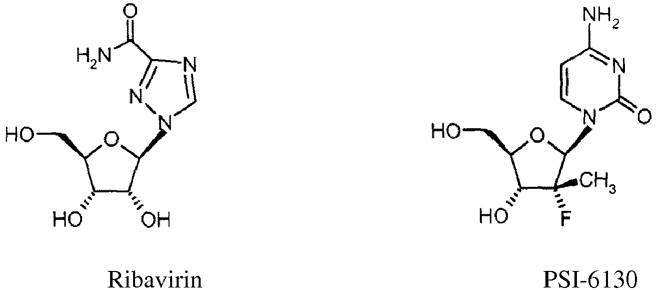

Adefovir (PMEA), tenofovir (PMPA) and PMPDAP are acyclic nucleoside phosphonate analogues with anti-HIV and anti-HBV activities.

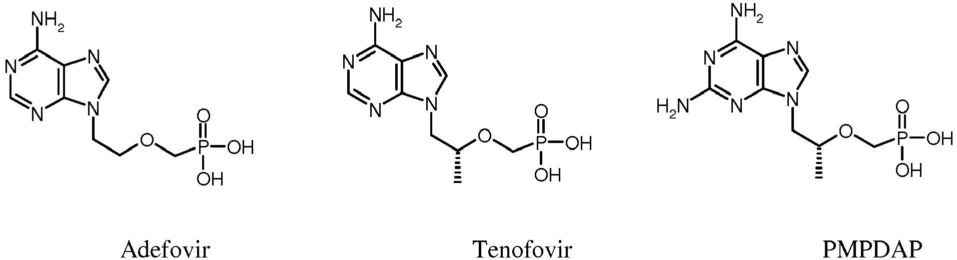

However, pharmacologically active molecules containing a phosphate or phosphonate group have difficulty in penetrating cytomembranes due to the strong polarity of the phosphate or phosphonate group, thus, the bioavailability of oral administration thereof is low and cannot achieve effective therapeutic concentrations. Therefore, the key to the development of such drugs is the research and development of safe and effective prodrugs of pharmacologically active molecules containing the phosphate or phosphonate group.

Making pharmaceutical molecules containing a phosphonate group into carboxylate or carbonate prodrugs can significantly improve the oral bioavailability of phosphonate drugs. Adefovir dipivoxil and LB-80380 are prodrugs of pivalate active ester, and tenofovir disoproxil is a prodrug of isopropyl carbonate. Adefovir dipivoxil and tenofovir disoproxil are the most widely used antiviral drugs in clinical applications.

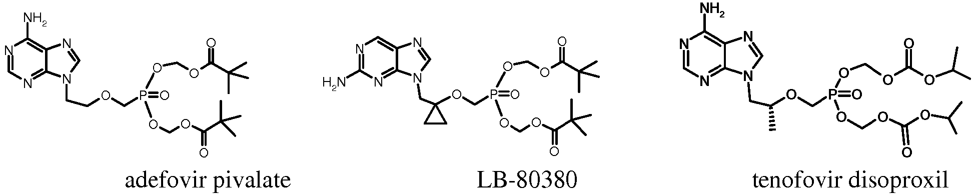

However, adefovir pivalate, LB-80380 and tenofovir disoproxil have poor chemical stability, and drugs substance and formulations thereof are more sensitive to temperature and humidity, and are prone to decomposing into monoesters which cannot be absorbed by human body; formaldehyde which is their metabolite *in vivo* has toxicity for human body; and due to their instability in gastrointestinal tract, they are easily hydrolyzed to generate highly acidic phosphonate compounds, thereby irritating the gastrointestinal tract. In addition, adefovir pivalate and LB-80380 enter into body and are hydrolyzed to generate pivalic acid, which is not easily metabolized and excreted and has a certain side-effect.

MCC-478 is a trifluoroethanol ester of acyclic nucleotide with anti-HBV virus effect and has better chemical stability. MCC-478 is hydrolyzed to release free acid (602076) after entering into the body, exerting antiviral effect; however, the research results on pharmacokinetics in phase I clinical trial suggest that the major metabolite of MCC-478 *in vivo* is nucleotide monoester (602074), and the blood concentration of the free acid 602076 is only one tenth of that of the monoester 602074 (Clark Chan, et al., Clinical Pharmaco-kinetics of Alamifovir and Its Metabolites Antivicrob Agents Chemother, 2005, 49(5):1813 - 1822.), and the cytotoxicity of the monoester 602074 (CC50 = 548 µM) is significantly higher than that of MCC-478 and 602076 (CC50 of both > 1000 µM) (Kamiya N, et al. Antiviral activities of MCC-478, a novel and specific inhibitor of hepatitis B Virus. Antimicrob Agents Chemother 2002 ;46(9):2872.).

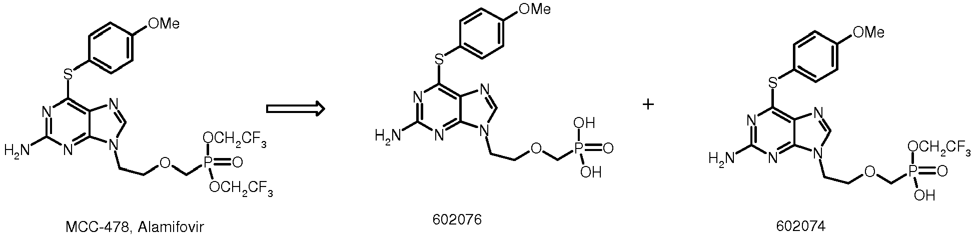

The design of S-acetylthioethylester (SATE) is also the most commonly used prodrug design strategy of phosphoric acid/phosphonic acid drugs. For example, the bis-(S-acetylthioethylester) of ddUMP and foscarnet are able to significantly enhance the oral bioavailability of ddUMP and foscarnet.

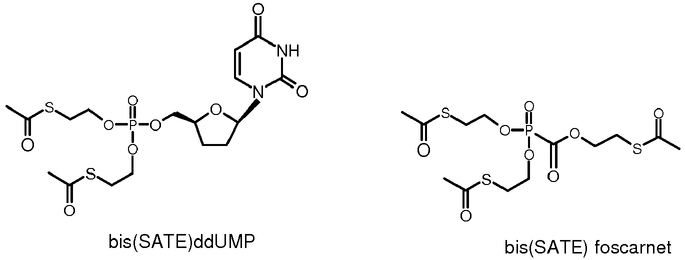

However, the S-acetylthioethylester (SATE) of phosphoric acid/phosphonic acid drugs enters into the body, generating vinyl sulfide which is a strong alkylation reagent.

CGS25463 and CGS26393 are diphenolic ester prodrugs of phosphonic acid derivatives, and can release active phosphonic acid drugs after entering into the body:

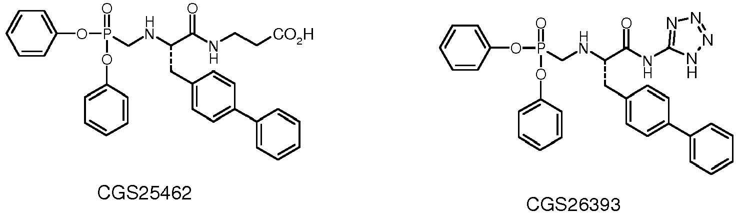

PSI-7977 and INX-08189 are prodrugs of phosphamide phenolic ester and phosphamide naphtholic ester of nucleotide with anti-HIV activity, respectively, release nucleoside monophosphate derivatives after entering into the body, and are further converted into triphosphate product, exerting anti-HCV effect:

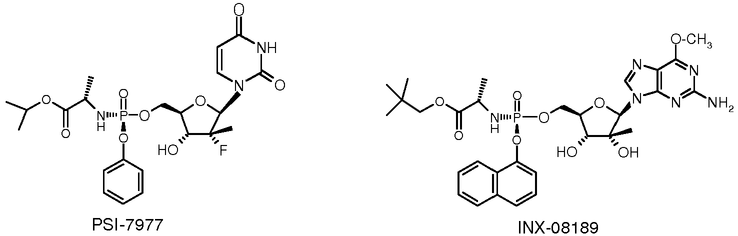

However, CGS25463, CGS26393, PSI-7977 and INX-08189 would generate highly toxic phenolic substances after entering into the body.

Pradefovir, MB07811 and MB07133 are prodrugs of benzene substituted cyclic phosphonates of anti-HBV drug PMEA, thyroid receptor agonist MB07344, and antineoplastic drug cytarabine, respectively; such prodrugs have better chemical stability, maintaining stable in the gastrointestinal tract and blood, and can be selectively oxidized by cytochrome P450 enzyme in the liver, and can targetedly release the active drug PMEA, MB07344 or cytarabine in the liver, exerting anti-HBV virus effect, hypolipidemic effect or anti-hepatocarcinoma effect:

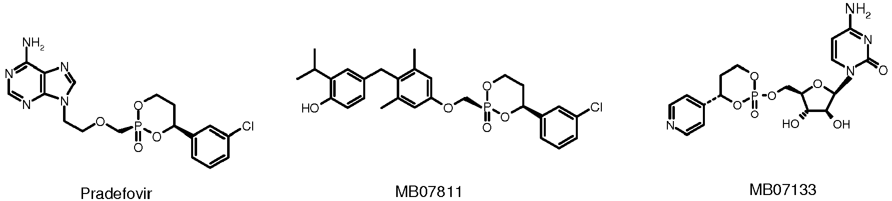

However, Pradefovir, MB07811 and MB07133 would generate metabolic intermediate with highly carcinogenic effects after entering into the body, and have carcinogenic effects.

Vinay et al. disclosed 2'-azido substituted nucleoside derivatives and use for the treatment of viral disease in WO2012142075A1, but didn't disclose the difference among the groups of phenyl and benzo[1,3]dioxolan-4-yl.

### Description of the Invention

To overcome the deficiencies of the foregoing phosphoric acid/phosphonic acid prodrugs, the present invention provides phosphoric acid/phosphonic acid derivatives shown in claim 1.

By a large number of research, the inventors found that phosphoric acid/phosphonic acid derivatives shown in claim 1 have higher bioactivity and better biosafety compared to the prior art; the inventors also unexpectedly found that the title compounds maintain stable in the gastrointestinal tract and blood, and can sufficiently release active substances in the liver and have better liver targeting property; the inventors further unexpectedly found that the title compounds shown in claim 1 have hepatoprotective effect after oral administration, and can inhibit liver damage caused by hepatitis viruses and rapidly lower the increased transaminase caused by hepatitis viruses.

Therefore, the present invention provides use of phosphoric acid/phosphonic acid derivatives shown in claim 1 and nontoxic, pharmaceutically acceptable salt thereof for the manufacture of a medicament for treating liver diseases such as viral hepatitis.

Another aspect of the present invention provides pharmaceutical compositions comprising phosphoric acid/phosphonic acid derivatives shown in claim 1 and nontoxic, pharmaceutically acceptable salts thereof as active ingredients and pharmaceutical excipients; these pharmaceutical compositions may be tablets, such as rapid-release tablets, sustained-release tablets, controlled-release tablets, film-coated tablets, sugar-coated tablets, buccal tablets, sublingual tablets, and the like; capsules such as hard capsules, soft capsules and the like; injections such as sterile or bacteriostat-containing aqueous injections, oily injections, freeze-dried powders for injection, microspheres for injection, and the like.

Phosphoric acid/phosphonic acid derivatives shown in claim 1 can be prepared according to the following synthetic schemes:

### Scheme 1: Taking the preparation of 5-hydroxylbenzodioxolane PMEA diester derivatives as an example, the title compound of phosphonates with both of R₁ and R₂ being Q2 or Q3 can be prepared in accordance with synthetic Scheme 1:

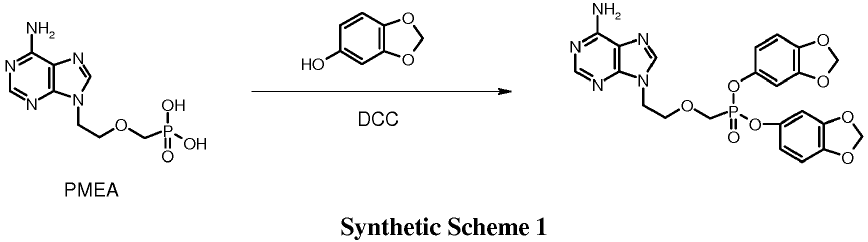

The title compound is obtained by the condensation of PMEA with the hydroxyl of 5-hydroxylbenzodioxolane under the action of dicyclohexylcarbodiimide.

### Scheme 2: Taking the preparation of 5-hydroxylbenzodioxolane phosphate derivatives of acyclovir as an example, the title compound of phosphates with both of R₁ and R₂ being Q2 or Q3 can be prepared in accordance with synthetic Scheme 2:

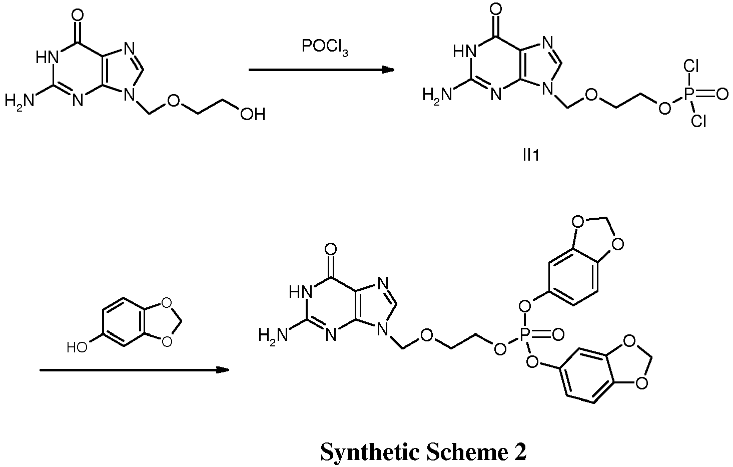

Acyclovir reacts with phosphorus oxychloride to generate phosphoryl chloride intermediate II₁, and then the II₁ is reacted with 5-hydroxylbenzodioxolane to obtain the title compound.

### Scheme 3: Taking the preparation of phosphonamide monoester derivatives of PMPA as an example, the title compound of phosphonamide monoesters with R₁ being Q1 and R₂ being Q2 or Q3 can be prepared in accordance with synthetic Scheme 3:

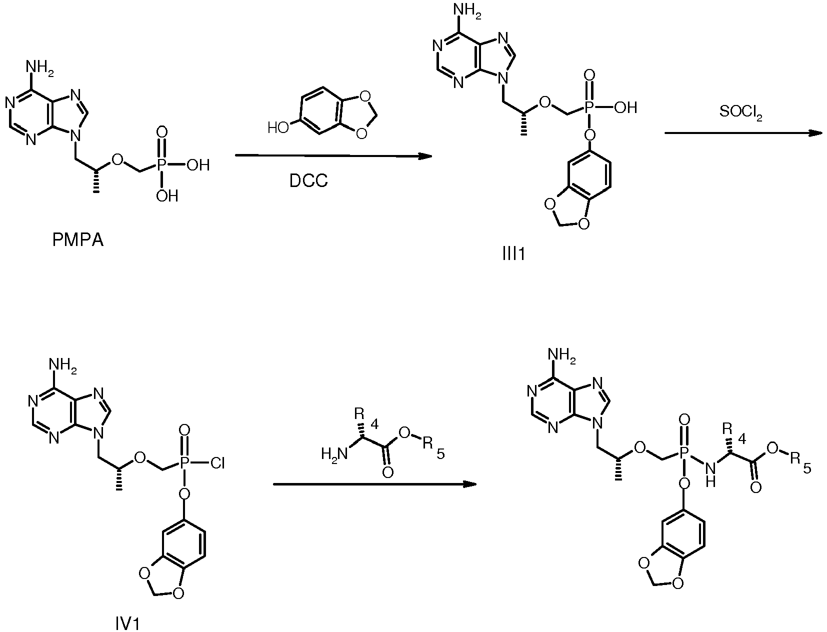

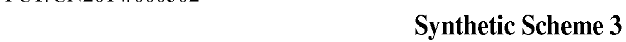

PMEA firstly is condensed with the hydroxyl of 5-hydroxylbenzodioxolane under the action of dicyclohexylcarbodiimide to obtain a monoester derivative III₁, and the III₁ reacts with sulfoxide chloride to generate a phosphonic chloride monoester intermediate IV₁, and the IV₁ is reacted with amino acid ester to obtain the title compound.

### Scheme 4: Taking the preparation of phosphoramide monoester derivatives of acyclovir as an example, the title compound of phosphoramide monoesters with R₁ being Q1 and R₂ being Q2 or Q3 can be prepared in accordance with synthetic Scheme 4:

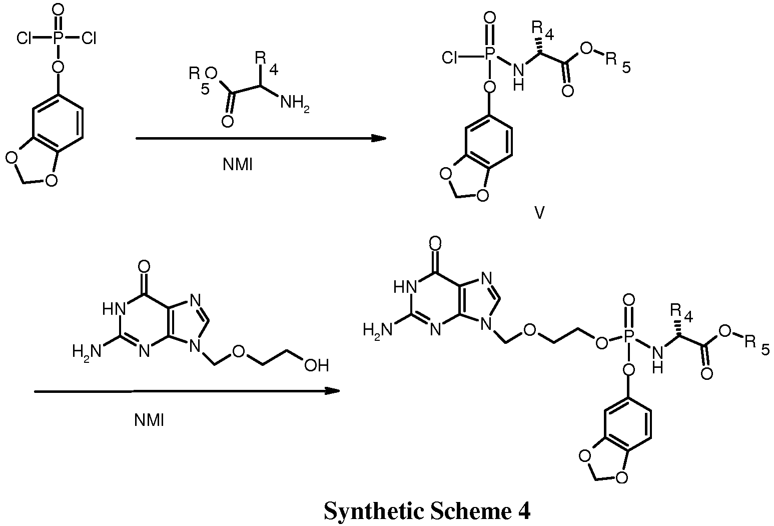

A phosphoester dichloride derivative is reacted with an amino acid derivative to obtain a chlorophosphoramide ester derivative V, and the V is reacted with the hydroxyl on the side chain of acyclovir to obtain the title compound.

### Detailed Description of Embodiments

The following examples are used to specifically explain the present invention, but the scope of the present invention is covered by the claims 1 to 4.

### Reference Example 1: Preparation of 9-[[bis-(phenoxyl)-phosphonyl-methoxyl]-ethyl]-adenine (i-1)

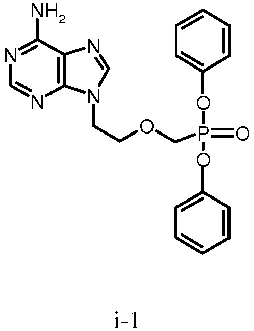

13 g of 9-[(phosphonyl-methoxyl)-ethyl]-adenine (PMEA) and 9.4 g of phenol were added to 100 ml of N-methylpyrrolidone and heated to 90 °C and stirred, and then 10 ml of triethylamine and 20 g of dicyclohexylcarbodiimide were added successively, heated and stirred at 90 °C for 15 hours. It was naturally cooled overnight, and the solid was filtered; the filtrate was concentrated under reduced pressure, the residue was dissolved in ethyl acetate and washed with the saturated solution of sodium carbonate (2X200 ml) and the saturated solution of sodium chloride (2X200 ml) successively, the organic layer was dried by anhydrous sodium sulfate overnight, the dessicant was then filtered, and the filtrate was evaporated under reduced pressure to dryness, the resulting residue was separated by silica gel column chromatography, eluted with a mixed solvent of dichloromethane:methanol (20:1), and the desired component was collected and evaporated under reduced pressure to dryness to obtain 4.1 g of i-1. H¹-NMR (ppm, DMSO-d6): 8.16 (s, 1H); 8.12 (s, 1H); 7.34-7.37 (m, 4H); 7.33 (b, 2H); 7.20-7.25 (m, 6H); 4.35-4.33 (t, 2H); 3.92-3.90 (t, 2H); 3.79-3.77 (d, 2H).

### Reference Example 2: Preparation of 9-[[bis-(naphthoxy)-phosphonyl-methoxyl]-ethyl]-adenine (i-2)

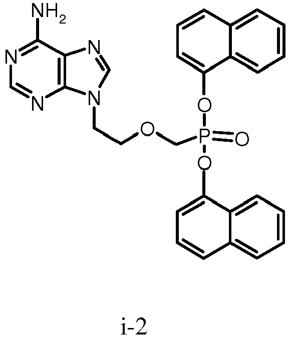

With reference to the method of Reference Example 1, naphthol was used instead of phenol to react with PMEA, the reaction product was purified by separation, to obtain i-2, with a yield of 27%. 8.16 (s, 1H); 8.12 (s, 1H); 7.73-7.77 (m, 2H); 7.45-7.59 (m, 10H); 7.20-7.25 (m, 6H); 6.22-6.34 (m, 2H); 4.35-4.33 (t, 2H); 3.92-3.90 (t, 2H); 3.79-3.77 (d, 2H).

### Reference Example 3: Preparation of 5'-[((phenoxyl-1-methoxylcarbonylethylamino)-phosphoryl]-2',3'-dideoxy-3'-thia-cytidine (Cf1109)

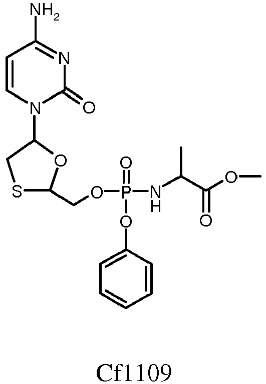

2.1 g (0.01 mol) of phenyl dichlorophosphate and 1.3 g (0.01 mol) of L-alanine methyl ester were dissolved in 30 ml of anhydrous dichloromethane, and cooled to -78°C. A solution of 2 ml of triethylamine dissolved in 20 ml of anhydrous dichloromethane was added dropwise with stirring, the rate of the dropwise adding was controlled to keep the reaction temperature at -78°C. After adding, when the reaction temperature was slowly raised to room temperature, stirring was continued for 1 hour. The solvent was evaporated under reduced pressure, and 30 ml of anhydrous diethyl ether was added to the residue and filtered. The filtrate was evaporated under reduced pressure to dryness to obtain a colourless oily matter, i.e., phosphoramide intermediate V₁, which was directly used in the next step reaction.

0.21 g of lamivudine was dissolved in 50 ml of THF, and then 7 ml of pyridine was added, and purged with nitrogen, 1 ml of 1M solution of tert-butyl magnesium chloride in THF was added and stirred for 0.5 h, and 2 ml of 1M solution of V₁ in THF was added with stirring and stirred for 2 hours. 50 ml of dichloromethane was added to the reaction solution, after being extracted with 100 ml of saturated aqueous solution of ammonium chloride, the organic layer was separated, and the aqueous layer was extracted with dichloromethane (3X50ml), the organic layer was combined, washed with a saturated aqueous solution of sodium chloride, and then dried by anhydrous magnesium sulfate overnight and filtered. The filtrate was evaporated under reduced pressure to dryness, the residue was separated by silica gel column chromatography, eluted with a mixed solvent of dichloromethane: methanol: triethylamine (100:5:1), and the desired component was collected and evaporated to dryness to obtain 0.18g of the title compound. H¹-NMR δ (ppm, DMSO-d6): 7.68 (d, 1H); 7.25-7.30 (m, 7H); 6.23 (t, 1H); 6.11 (m, 1H); 5.72(d ,1H); 5.35 (t ,1 H); 4.27 (m, 2H); 3.88 (m, 1H); 3.58 (s ,3H); 3.42 (q , 1H); 3.05 (m, 2H); 1.21 (d, 3H).

### Reference Example 4: Preparation of 9-[(R)-2-[[(S)-[[(S)-1-(isopropoxycarbonyl)-ethyl]-amino] -phenoxylphosphonyl] -methoxyl] -propyl] -adenine fumarate (GS-7340)

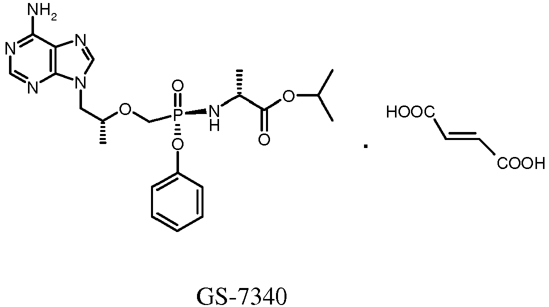

14.6 g of 9-[(R)-2-(phosphonylmethoxyl)-propyl]-adenine (PMPA) and 9.6 g of phenol were added to 50 ml of N-methylpyrrolidone and heated to 85 °C, and then 6.3ml of triethylamine was added. 13.4g of DCC was slowly added under stirring and heated at 100 °C and stirred overnight, and 30 ml of water was added after cooling. After standing, the solid was filtered, and the filtrate was combined and evaporated under reduced pressure to dryness, 30 ml of water was added, and the pH was adjusted to 11 with 25% sodium hydroxide, the solid was filtered, and the filtrate was combined and extracted by 30ml of ethyl acetate. The pH of the aqueous solution was adjusted to 3.1 by 37% hydrochloric acid, it was left and the solid was precipitated out. The solid was collected by filtration, and then washed by adding 50 ml of methanol under stirring, filtered and dried under vacuum, to obtain 7.2g of phenol monoester derivatives of PMPA.

7.1 g of product from the last step was added to 30 ml of acetonitrile, 6.2g of sulfoxide chloride was added dropwise with stirring, and the temperature was kept below 50°C. The reaction mixture was heated at 75°C until the solid was dissolved, and then heated to 80°C and evaporated to dryness. Cooling to 25°C, 40 ml of dichloromethane was added and cooled to -30 °C; 6.5 g of L-alanine isopropyl ester was added to 35 ml of dichloromethane solution over 30 minutes and the temperature was kept at -18°C. Then, 7 ml of triethylamine was added dropwise over 15 minutes and the temperature was kept from -18°C to -11°C. The mixture was stirred at room temperature for 2 hours, and washed with 10% sodium dihydrogen phosphate solution (3X15 ml). The organic layer was dried by anhydrous sodium sulfate, and after filtering, the filtrate was evaporated under reduced pressure to dryness, the residue was separated by silica gel column chromatography, eluted with acetone, and the desired component was collected and evaporated under reduced pressure to dryness to obtain a 3.6 g of oily matter. The resulting oily matter was separated by chiral preparative chromatography (Diacel's Chiralpak AS), eluted with acetonitrile containing 30% methanol, the second main peak was collected and evaporated under reduced pressure to dryness to obtain 1.3 g of brownish yellow solid. 25 ml of acetonitrile and 0.3 g of fumaric acid were added, heated to reflux until the solid was dissolved and filtered immediately. The filtrate was cooled to 5°C, and left to stand overnight. Then the filtrate was filtered and dried, to obtain 1.2 g of white solid, with the melting point of 120-122°C, [α]D²⁰ -41.7°(c 1.0, acetic acid).

### Reference Example 5: Preparation of 5'-[(S)-[[(S)-1-(isopropoxycarbonyl)-ethyl]-amino]-phenoxylphosphoryl]-β-D-2'-deoxy-2'-α-fluoro-2'-β-C-methyluridine (PSI-7977)

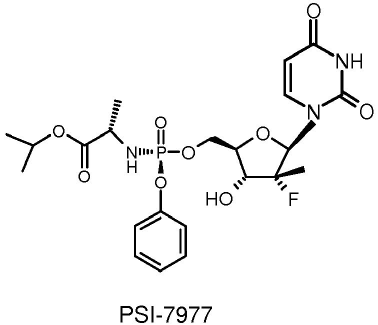

2.1 g (0.01 mol) of phenoxyl-phosphorus oxydichloride and 1.6 g (0.01 mol) of L-alanine isopropyl ester were dissolved in 30 ml of anhydrous dichloromethane, and cooled to -78°C. A solution of 2ml of triethylamine in 20 ml of anhydrous dichloromethane was added dropwise with stirring, the rate of the dropwise adding was controlled to keep the reaction temperature at -78°C. After adding, when the reaction temperature was slowly raised to room temperature, stirring was continued for 1 hour. The solvent was evaporated under reduced pressure, and 30 ml of anhydrous diethyl ether was added to the residue and then filtered. The filtrate was evaporated under reduced pressure to dryness to obtain a colourless oily matter, i.e., phosphoramide intermediate V₂, which was directly used in the next step reaction.

1.5g of β-D-2'-deoxy-2'-α-fluoro-2'-β-C-methyluridine (Ji'nan Branch of A Chemical Co., Ltd., the purity of 98%) was dissolved in 30 ml of THF, and then 3 g of N-methylimidazole was added, and a solution of V₂ in 20 ml of THF was added. It was stirred at room temperature overnight, filtered, and the filtrate was evaporated under reduced pressure to dryness. The residue was separated by silica gel column chromatography, eluted with dichloromethane containing 2% isopropanol, and the desired component was collected and evaporated under reduced pressure to dryness. The residue was dissolved with acetonitrile containing 20% isopropanol and separated by chiral preparative chromatography with the chromatography column of Diacel's Chiralpak AS, the mobile phase being acetonitrile solution containing 20 % isopropanol, the flow rate being 8 ml/min, the second component was collected and evaporated under reduced pressure to dryness, to obtain 0.21g of PSI-7977. H¹-NMR δ (ppm, DMSO-d6): 11.21 (s, 1H); 7.52 (d, 1H); 7.36-7.30 (m, 2H); 7.20-7.12 (m, 3H); 6.05-5.95(m, 2H); 5.80 (m, 1H); 5.51 (d, 1H); 4.81 (q, 1H); 4.36-4.30 (m, 1H); 4.21-4.16 (m, 1H); 4.00-3.94 (m, 1H); 3.82-3.70 (m, 2H); 1.21 (d, 3H); 1.18 (d,3H); 1.10 (d, 6H).

### Reference Example 6: Preparation of 5'-[((phenoxyl-1-isopropoxycarbonylethylamino)-phosphoryl]-β-D-2'-deoxy-2'-α-fluoro-2'-β-C-methyl-6-O-methylguanosine(PSI-353661)

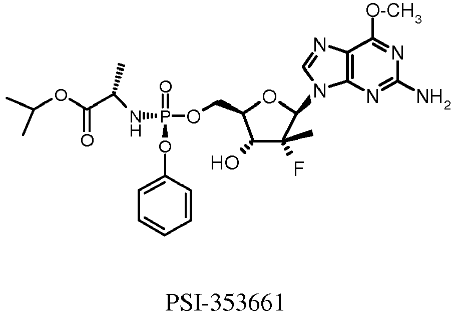

With reference to the method of Reference Example 5, β-D-2'-deoxy-2'-α-fluoro-2'-β -C-methyl-6-O-methylguanosine (Ji'nan Branch of A Chemical Co., Ltd., the purity of 98%) was used instead of β-D-2'-deoxy-2'-α-fluoro-2'-β-C-methyluridine to react with phosphoramide intermediate V₂, and after treatment, separation and purification by similar methods, the title compound PSI-353661 was obtained. H¹-NMR δ (ppm, DMSO-d6): 10.70 (s, 1H); 7.38-7.32 (m, 2H); 7.22-7.15 (m, 3H); 6.52 (s,2H); 6.08-5.92 (m, 3H); 5.82 (m, 1H); 4.84 (q, 1H); 4.39-4.33 (m, 1H); 4.25-4.19 (m, 1H); 4.03-3.97m, 1H); 3.91 (s,3H); 3.85-3.73 (m, 2H); 1.24 (d, 3H); 1.22 (d,3H); 1.12(d, 6H).

### Reference Example 7: Preparation of 9-[[[(phenoxyl-1-methoxylcarbonylethylamino)-phosphoryl]-oxyl]-ethoxyl]-methyl]-guanine (i-3)

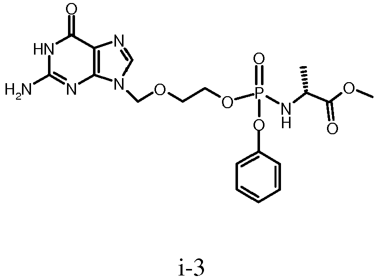

With reference to the method of Reference Example 3, acyclovir was used instead of lamivudine to react with phosphoramide intermediate V₁, and after treatment, separation and purification by similar methods, the title compound i-3 was obtained. H¹-NMR (ppm, DMSO-d6): 10.65 (s, 1H); 7.83 (s, 1H); 7.38-7.14 (m, 5H); 6.53 (s, 2H); 6.00-5.93 (m, 1H); 5.36 (s, 2H); 4.13-4.05 (m, 2H); 3.85-3.80 (m, 1H); 3.72-3.63 (m, 2H); 3.59 (s, 3H); 1.23-1.19 (m, 3H).

### Reference Example 8: Preparation of 2-amino-1,9-dihydro-9-[(1S,3R,4S)-4-hydroxyl-3-((R)-((S)-1-ethoxylcarbonylethylamino-phenoxy-phosphoryl)-oxymethyl)-2-methylenecyclopentyl]-6H-purin-6-one (i-4) and 2-amino-1,9-dihydro-9-[(1S,3R,4S)-4-hydroxyl-3-((S)-((S)-1-isopropoxycarbonylethylamino-phenoxy-phosphoryl)-oxymethyl)-2-methylenecyclopentyl]-6H-purin-6-one (i-5)

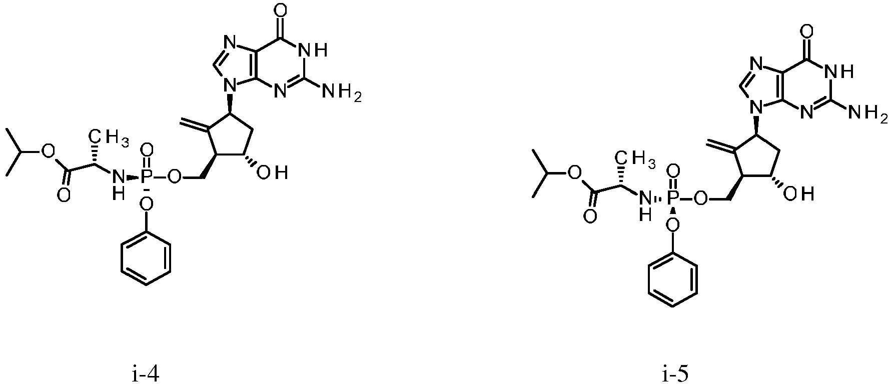

5.5 g of anhydrous entecavir was dissolved in 50 ml of THF, and then 6 g of N-methylimidazole was added, and a solution of 6.3 g of V₂ dissolved in 30 ml of THF was added. It was stirred at room temperature overnight, filtered, and the filtrate was evaporated under reduced pressure to dryness. The residue was separated by silica gel column chromatography, eluted with dichloromethane containing 2% isopropanol, the desired component was collected and evaporated under reduced pressure to dryness. The residue was dissolved with acetonitrile containing 20% isopropanol and separated by chiral preparative chromatography, with the chromatography column of Diacel's Chiralpak AS, the mobile phase being acetonitrile solution containing 20 % isopropanol, the flow rate being 8 ml/min, the first component was collected and evaporated under reduced pressure to dryness to obtain 0.20 g of i-4; the second component was collected and evaporated under reduced pressure to dryness to obtain 0.24 g of i-5.

H¹-NMR of i-4 δ (ppm, DMSO-d6 ,400MHz): 10.51(bs, 1H); 7.63(s, 1H); 7.39-7.35 (m, 2H); 7.22-7.17 (m, 3H); 6.09 (m, 1H); 6.45 (s, 2H); 5.32 (m, 1H); 5.06(m, 1H); 4.89 (d, 1H); 4.85-4.83 (m, 2H); 4.52 (m, 1H); 4.40 (m, 1H); 4.23 (m, 1H); 3.51(m, 2H); 2.52 (m, 1H); 2.22 (m, 1H); 2.04 (m, 1H); 1.19 (d, 3H); 1.16 (d , 6H).

H¹-NMR of i-5 δ (ppm, DMSO-d6 ,400MHz) : 10.54(bs, 1H); 7.67(s, 1H); 7.38-7.33 (m, 2H); 7.21-7.15 (m, 3H); 6.45 (s, 2H); 6.03 (m, 1H); 5.80 (m, 1H); 5.32 (m, 1H); 5.07(m, 1H); 4.87-4.83 (m, 3H); 4.58 (m,1H); 4.35 (m, 1H); 4.23 (m, 1H); 3.51 (m, 2H); 2.50 (m,1H); 2.21 (m, 1H); 2.03 (m, 1H); 1.21 (d, 3H); 1.13 (d, 6H).

### Example 1: Preparation of 9-[[[bis-((benzo[1,3]dioxolan-4-yl)-oxyl)-phosphonyl]-methoxyl]-ethyl]-adenine (I₁)

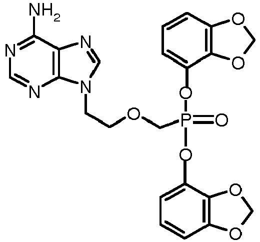

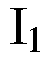

13.7g of 9-[(phosphonyl-methoxyl)-ethyl]-adenine (PMEA) and 13.8g of 4-hydroxylbenzo[1,3]dioxolane were added to 100ml of N-methylpyrrolidone, heated to 90 °C and stirred, and then 10ml of triethylamine and 20g of dicyclohexylcarbodiimide were added successively, heated and stirred at 90 °C for 15 hours. It was naturally cooled overnight, and the solid was filtered; the filtrate was concentrated under reduced pressure, the residue was dissolved in ethyl acetate and washed with the saturated solution of sodium carbonate (2X200 ml) and the saturated solution of sodium chloride (2X200 ml) successively, the organic layer was dried by anhydrous sodium sulfate overnight, the dessicant was then filtered, the filtrate was evaporated under reduced pressure to dryness, and the resulting residue was then separated by silica gel column chromatography, eluted with a mixed solvent of dichloromethane: methanol (20: 1), and the desired component was collected and evaporated under reduced pressure to dryness to obtain 3.2g of I₁. H¹-NMR δ (ppm, DMSO-d6): 8.16 (s, 1H), 8.12 (s, 1H), 7.33 (b, 2H), 6.67-6.71 (t, 2H); 6.58-6.60 (d, 2H); 6.53-6.55 (d, 2H); 5.97-5.98 (d, 4H), 4.35-4.33 (t, 2H), 3.92-3.90 (t, 2H), 3.79-3.77 (d, 2H).

### Example 2: Preparation of 9-[[[bis-((benzo[1,3]dioxolan-5-yl)-oxyl)-phosphonyl]-methoxyl]-ethyl]-adenine (I₂)

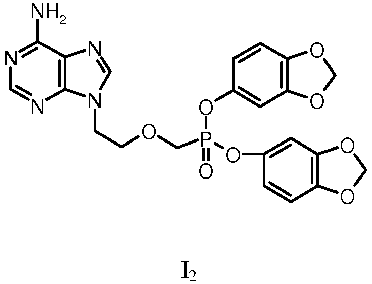

With reference to the method of Example 1, 5-hydroxylbenzo[1,3]dioxolane was used instead of 4-hydroxylbenzo[1,3]dioxolane to be condensed with PMEA, and after treatment, separation and purification by similar methods, the title compound I₂ was obtained, with a yield of 14%. H¹-NMR δ (ppm, DMSO-d6): 8.15 (s, 1H); 8.11 (s, 1H); 7.32 (b, 2H); 6.77-6.75 (d, 2H); 6.68 (s, 2H); 6.49-6.47 (d, 2H); 5.99 (s, 4H); 4.35-4.33 (t, 2H); 3.93-3.91 (t, 2H); 3.80-3.78 (d, 2H).

### Example 3: Preparation of 9-[[[bis-((benzo[1,4]dioxan-5-yl)-oxyl)-phosphonyl]-methoxyl] -ethyl]-adenine (I₃)

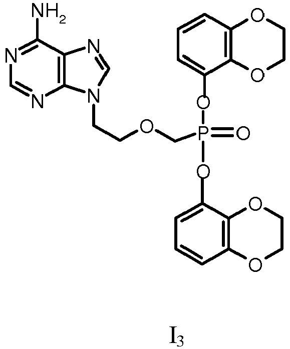

With reference to the method of Example 1, 5-hydroxylbenzo[1,4]dioxane was used instead of 4-hydroxylbenzo[1,3]dioxolane to be condensed with PMEA, and after treatment, separation and purification by similar methods, the title compound I₃ was obtained, with a yield of 19%. H¹-NMR δ (ppm, DMSO-d6): 8.16 (s, 1H); 8.12 (s, 1H); 7.33 (b, 2H); 6.58-6.62 (t, 2H); 6.51-6.53 (d, 2H); 6.41-6.43 (d, 2H); 4.35-4.33 (t, 2H); 4.20-4.24 (m, 8H); 3.92-3.90 (t, 2H); 3.79-3.77 (d, 2H).

### Example 4: Preparation of 9-[[[bis-((benzo[1,4]dioxan-6-yl)-oxyl)-phosphonyl]-methoxyl] -ethyl]-adenine (I₄)

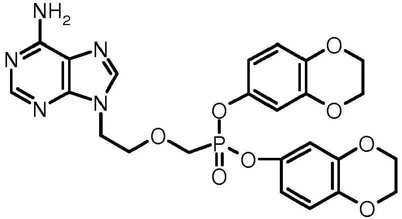

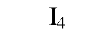

With reference to the method of Example 1, 5-hydroxylbenzo[1,4]dioxane was used instead of 4-hydroxylbenzo[1,3]dioxolane to be condensed with PMEA, and after treatment, separation and purification by similar methods, the title compound I₄ was obtained, with a yield of 14%. H¹-NMR δ (ppm, DMSO-d6): 8.16 (s, 1H); 8.12 (s, 1H); 7.33 (b, 2H); 6.77-6.75 (d, 2H); 6.68 (s,2H); 6.49-6.47 (d, 2H); 4.35-4.33 (t, 2H); 4.20-4.24 (m, 8H); 3.92-3.90 (t, 2H); 3.79-3.77 (d, 2H).

### Example 5: Preparation of 9-[(R)-2-[[bis-((benzo[1,3]dioxolan-4-yl)-oxyl)-phosphonyl]-methoxyl]-propyl]-adenine (I₅)

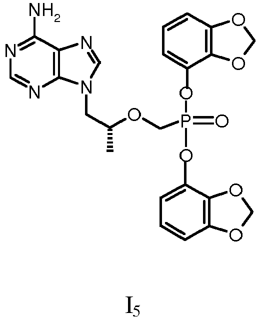

With reference to the method of Example 1, 4-hydroxylbenzo[1,3]dioxolane was condensed with 9-(R)-[2-(phosphonomethoxyl)-propyl]-adenine (R-PMPA), and after treatment, separation and purification by similar methods, the title compound I₅ was obtained, with a yield of 23%. H¹-NMR δ (ppm, DMSO-d6): 8.08 (s, 1H); 8.07 (s, 1H); 7.60 (b, 2H); 6.67-6.71 (t, 2H); 6.58-6.60 (d, 2H); 6.53-6.55 (d, 2H); 5.97-5.98 (s, 4H); 4.41 (m, 1H); 3.92-3.90 (d, 2H); 3.79-3.77 (d, 2H); 1.29 (d,3H).

### Example 6: Preparation of 9-[(R)-2-[[bis-((benzo[1,3]dioxolan-5-yl)-oxyl)-phosphonyl] -methoxyl]-propyl]-adenine (I₆)

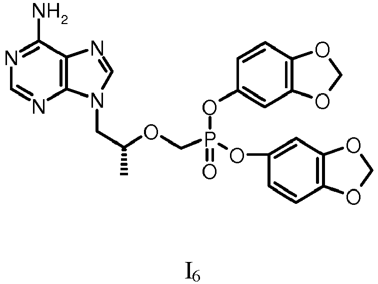

With reference to the method of Example 1, 5-hydroxylbenzo[1,3]dioxolane was condensed with R-PMPA, and after treatment, separation and purification by similar methods, the title compound I₆ was obtained, with a yield of 19%. H¹-NMR δ (ppm, DMSO-d6): 8.08 (s, 1H); 8.07 (s, 1H); 7.33 (b, 2H); 6.77-6.75 (d, 2H); 6.68 (s,2H); 6.49-6.47 (d, 2H); 5.99 (s, 4H); 4.41 (m, 1H); 3.92-3.90 (d, 2H); 3.79-3.77 (d, 2H); 1.29 (d,3H).

### Example 7: Preparation of 2-amino-6-(4-methoxylphenyl)thio-9-[[[bis-((benzo[1,3]dioxolan-4-yl)-oxyl)-phosphonyl]-methoxyl]-ethyl] -purine (I₇)

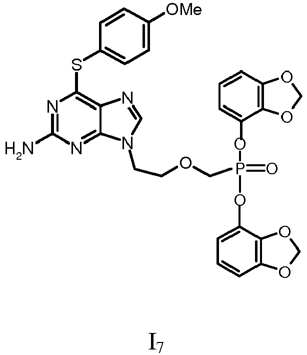

With reference to the method of Example 1, 4-hydroxylbenzo[1,3]dioxolane was condensed with 2-amino-6-(4-methoxylphenyl)thio-9-[(phosphonyl-methoxyl)-ethyl]-purine, and after treatment, separation and purification by similar methods, the title compound I₇ was obtained, with a yield of 14%. H¹-NMR δ (ppm, DMSO-d6): 7.89 (s,1H); 7.54 (d, 2H); 7.32 (b, 2H); 6.91 (d, 2H); 6.67-6.71 (t, 2H); 6.58-6.60 (d, 2H); 6.53-6.55 (d, 2H); 5.97-5.98 (s, 4H); 4.35-4.33 (t, 2H); 3.93-3.91 (t, 2H); 3.89 (s, 3H); 3.80-3.78 (d, 2H).

### Example 8: Preparation of 2-amino-6-(4-methoxylphenyl)thio-9-[[[bis-((benzo[1,3]dioxolan-5-yl)-oxyl)-phosphonyl]-methoxyl]-ethyl]-purine (I₈)

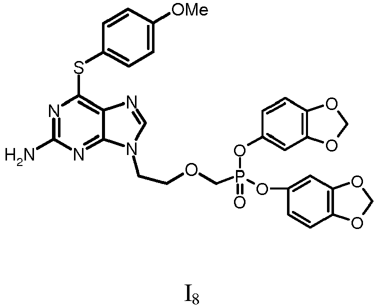

With reference to the method of Example 1, 5-hydroxylbenzo[1,3]dioxolane was condensed with 2-amino-6-(4-methoxylphenyl)thio-9-[(phosphonyl-methoxyl)-ethyl]-purine, and after treatment, separation and purification by similar methods, the title compound I₈ was obtained, with a yield of 16%. H¹-NMR δ (ppm, DMSO-d6): 7.89 (s,1H); 7.54 (d,2H); 7.32 (b, 2H); 6.91 (d,2H); 6.77-6.75 (d, 2H); 6.68(s, 2H); 6.49-6.47 (d, 2H); 5.99 (s, 4H); 4.35-4.33 (t, 2H); 3.93-3.91 (t, 2H); 3.89 (s, 3H); 3.80-3.78 (d, 2H).

### Example 9: Preparation of 9-[[[bis-((benzo[1,3]dioxolan-4-yl)-oxyl)-phosphoryloxyl]-ethoxyl]-methyl]-guanine (I₁₁)

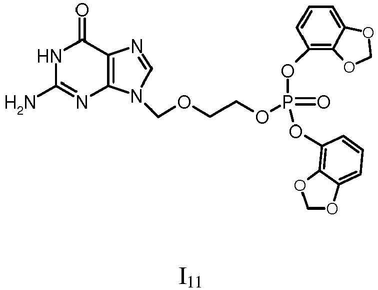

4.5 g of anhydrous acyclovir was added to 100 ml of dry THF, and purged with nitrogen, and the reaction mixture was stirred and cooled to 15°C to -20°C. A solution of 2.8 g of phosphorus oxychloride and 8 ml of anhydrous dichloromethane was added dropwise with stirring, and the temperature of the reaction mixture was kept from -15°C to -20°C, stirring was continued until acyclovir was completely reacted, and the reaction solution was evaporated under reduced pressure to dryness to obtain dichlorophosphate derivatives II₁ of acyclovir.

The II₁ was dissolved with 50 ml of anhydrous dichloromethane and cooled to -78 °C, and then a solution of 8.3 g of 4-hydroxylbenzo[1,3]dioxolane and 6 g of freshly distilled triethylamine in 50 ml of anhydrous dichloromethane were added dropwise with stirring. After adding, it was stirred, gradually raised to room temperature and stirred overnight. After filtering, the filtrate was evaporated under reduced pressure to dryness, the residue was dissolved with ethyl acetate and washed with the saturated solution of sodium carbonate (2X200 ml) and the saturated solution of sodium chloride (2X200 ml) successively, the organic layer was dried by anhydrous sodium sulfate overnight, the dessicant was then filtered, the filtrate was evaporated under reduced pressure to dryness, and then separated by silica gel column chromatography, eluted with a mixed solvent of dichloromethane: methanol (20 : 1), and the desired component was collected and evaporated under reduced pressure to dryness to obtain 1.2g of I₁₁. H¹-NMR (ppm, DMSO-d6): 10.65 (s, 1H); 7.81 (s, 1H); 6.67-6.71 (t, 2H); 6.58-6.60 (d, 2H); 6.53-6.55 (d, 2H); 6.5 (bs, 2H); 5.35 (s, 2H); 5.97-5.98 (s, 4H); 4.1-4.0 (t, 2H); 3.7-3.6 (t, 2H).

### Example 10: Preparation of 9-[[[bis-((benzo[1,3]dioxolan-5-yl)-oxyl)-phosphoryloxyl]-ethoxyl]-methyl]-guanine (I₁₂)

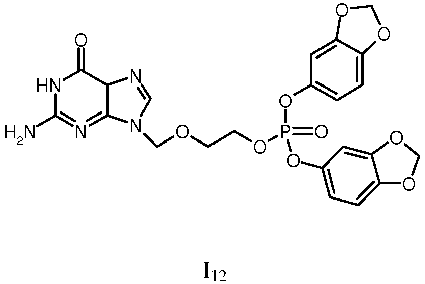

With reference to the method of Example 9, 5-hydroxylbenzo[1,3]dioxolane was used instead of 4-hydroxylbenzo[1,3]dioxolane to react with II₁, and after treatment, separation and purification by similar methods, the title compound I₁₂ was obtained, with a yield of 17%. H¹-NMR (ppm, DMSO-d6): 10.65 (s, 1H); 7.81 (s, 1H); 6.77-6.75 (d, 2H); 6.68 (s, 2H); 6.5 (bs, 2H); 6.49-6.47 (d, 2H); 5.99 (s, 4H); 5.35 (s, 2H); 4.1-4.0 (t, 2H); 3.7-3.6 (t, 2H).

### Example 11: Preparation of 2-amino-6-methoxyl-9-[[[bis-((benzo[1,3]dioxolan-5-yl)-oxyl)-p hosphoryloxyl]-ethoxyl]-methyl]-purine (I₁₃)

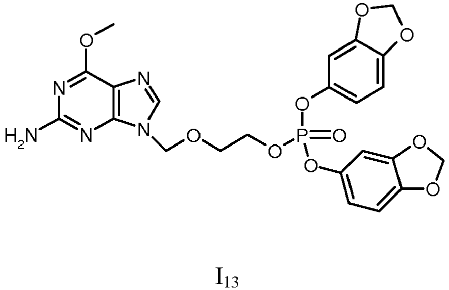

With reference to the method of Example 9, 2-amino-6-methoxyl-9-[(2-hydroxylethoxyl)-methyl]-purine was used instead of acyclovir to react with phosphorus oxychloride, and after treatment, separation and purification by similar methods, 2-amino-6-methoxyl-9-[(2-dichlorophosphoryloxyl-ethoxyl)-methyl]-purine (II₂) was obtained.

5-hydroxylbenzo[1,3]dioxolane was used to react with the II₂, and after treatment, separation and purification by similar methods, the title compound I₁₃ was obtained, with a yield of 20%. H¹-NMR (ppm, DMSO-d6): 7.98 (s, 1H); 6.77-6.75 (d, 2H); 6.68 (s, 2H); 6.5 (bs, 2H); 6.49-6.47 (d, 2H); 5.99 (s, 4H); 5.35 (s, 2H); 3.91 (s, 3H); 4.1-4.0 (t, 2H); 3.7-3.6 (t, 2H).

### Example 12: Preparation of 5'-bis-[((benzo[1,3]dioxolan-5-yl)-oxyl)-phosphoryl]-2',3'-dideoxy -3'-thia-cytidine (I₁₄)

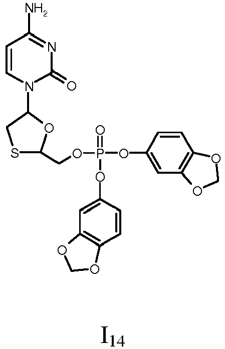

With reference to the method of Example 9, lamivudine was used instead of acyclovir to react with phosphorus oxychloride, and after treatment, separation and purification by similar methods, dichlorophosphate derivatives II₃ of lamivudine was obtained.

5-hydroxylbenzo[1,3]dioxolane was used to react with the II₃, and after treatment, separation and purification by similar methods, the title compound I₁₄ was obtained, with a yield of 12.0%. H¹-NMR (ppm, DMSO-d6): 7.71 (d, 1H); 7.30 (b, 2H); 6.77-6.75 (d, 2H); 6.68 (s, 2H); 6.49-6.47 (d, 2H); 6.25 (t, 1H); 5.99 (s, 4H); 5.74 (d, 1H); 5.38 (t, 1H); 4.29 (m , 2H); 3.89 (m, 1H); 3.06 (m, 2H).

### Example 13: Preparation of 9-[(1S,3R,4S)-4-hydroxyl-3-(bis-(((benzo[1,3]dioxolan-5-yl) -oxyl)-phosphoryl)-oxymethyl)-2-methylenecyclopentyl]-guanine (I₁₅)

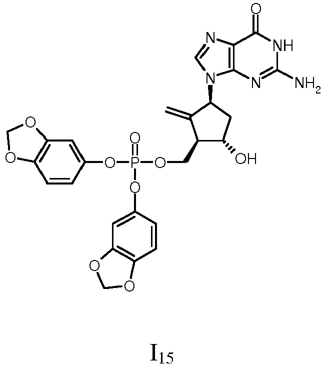

With reference to the method of Example 9, entecavir was used instead of acyclovir to react with phosphorus oxychloride, and after treatment, separation and purification by similar methods, dichlorophosphate derivatives II₄ of entecavir was obtained.

5-hydroxylbenzo[1,3]dioxolane was used to react with the II₄, and after treatment, separation and purification by similar methods, the title compound I₁₅ was obtained, with a yield of 23%. H¹-NMR (ppm, DMSO-d6): 10.54 (bs, 1H); 7.66 (s, 1H); 6.77-6.75 (d, 2H); 6.68 (s, 2H); 6.49-6.47 (d, 2H); 6.42 (s, 2H); 5.99 (s, 4H); 5.36 (m, 1H); 5.10 (m, 1H); 4.87 (d, 1H); 4.84 (m, 1H); 4.56 (m, 1H); 4.23 (m, 1H); 3.53 (m, 2H); 2.52 (m,1H); 2.22 (m, 1H); 2.04 (m, 1H).

### Example 14: Preparation of 2-amino-6-methoxyl-9-[(1S,3R,4S)-4-hydroxyl-3-(bis-(((benzo[1,3] dioxolan-5-yl)-oxyl)-phosphoryl)-oxymethyl)-2-methylenecyclopentyl]-purine (I₁₆)

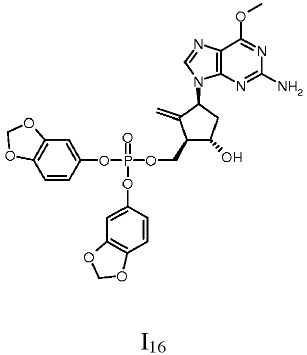

With reference to the method of Example 9, 2-amino-6-methoxyl-9-[(1S,3R,4S)-4-hydroxyl-3-hydroxymethyl)-2-methylenecyclopentyl]-purine was used instead of acyclovir to react with phosphorus oxychloride, and after treatment, separation and purification by similar methods, 2-amino-6-methoxyl-9-[(1S,3R,4S)-4-hydroxyl-3-(dichlorophosphoryloxymethyl)-2-methylenecyclopentyl]-purine(II₅) was obtained.

5-hydroxylbenzo[1,3]dioxolane was used to react with the II₅, and after treatment, separation and purification by similar methods, the title compound I₁₅ was obtained, with a yield of 15%. H¹-NMR (ppm, DMSO-d6): 7.96 (s, 1H); 6.77-6.75 (d, 2H); 6.68 (s, 2H); 6.49-6.47 (d, 2H); 6.42 (s, 2H); 5.99 (s, 4H); 5.36 (m, 1H); 5.10 (m, 1H); 4.87 (d, 1H); 4.84 (m, 1H); 4.56 (m,1H); 4.23 (m, 1H); 3.94 (s, 3H); 3.53(m, 2H); 2.52 (m,1H); 2.22 (m, 1H); 2.04 (m, 1H).

### Example 15: Preparation of 5'-O-[bis-((benzo[1,3]dioxolan-5-yl)-oxyl)-phosphoryl]-β-D-2'-d eoxy-2'-α-fluoro-2'-β-C-methyluridine (I₁₇)

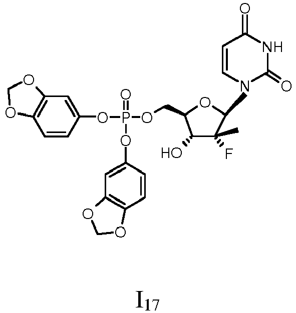

With reference to the method of Example 9, 2-amino-6-methoxyl-9-[(1S,3R,4S)-4-hydroxyl-3-hydroxymethyl)-2-methylenecyclopentyl]-purine was used instead of acyclovir to react with phosphorus oxychloride, and after treatment, separation and purification by similar methods, 2-amino-6-methoxyl-9-[(1S,3R,4S)-4-hydroxyl-3-(dichlorophosphoryloxymethyl)-2-methylenecyclopentyl]-purine (II₆) was obtained.

5-hydroxylbenzo[1,3]dioxolane was used to react with the II₆, and after treatment, separation and purification by similar methods, the title compound I₁₅ was obtained, with a yield of 12.5%. H¹-NMR (ppm, DMSO-d6): 11.37 (s, 1H); 7.82 (d, 1H); 6.77-6.75 (d, 2H); 6.68 (s,2H); 6.49-6.47 (d, 2H); 5.99 (s, 4H); 5.95 (d, 1H); 5.51 (d, 1H); 3.82-3.70 (m, 3H); 3.63-3.60 (m, 1H); 1.21 (d, 3H).

### Example 16: Preparation of 5'-O-[bis-((benzo[1,3]dioxolan-5-yl)-oxyl)-phosphoryl]-β-D-2'-d eoxy-2'-α-fluoro-2'-β-C-methyl-6-O-methylguanosine (I₁₈)

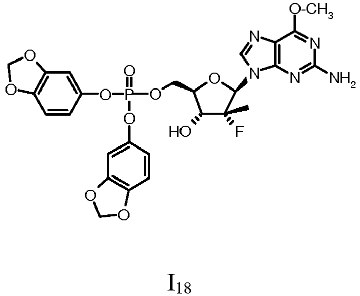

With reference to the method of Example 9, 5'-O-phosphoryl-β-D-2'-deoxy-2'-α-fluoro-2'-β-C-methyl-6-O-methylguanosine was used instead of acyclovir to react with phosphorus oxychloride, and after treatment, separation and purification by similar methods, 5'-O-(dichlorophosphoryl)-β-D-2'-deoxy-2'-α-fluoro-2'-β-C-methyl-6-O-methylguanosine (II₇) was obtained.

5-hydroxylbenzo[1,3]dioxolane was used to react with the II₇, and after treatment, separation and purification by similar methods, the title compound I₁₅ was obtained, with a yield of 13.9%. H¹-NMR (ppm, DMSO-d6): 7.98 (s, 1H); 6.77-6.75 (d, 2H); 6.68 (s, 2H); 6.52 (s, 2H); 6.49-6.47 (d, 2H); 6.08-5.92 (m, 6H); 3.91 (s, 3H); 3.85-3.73 (m, 2H); 1.25 (d, 3H).

### Example 17: Preparation of 9-[(R)-2-[(((benzo[1,3]dioxolan-5-yl)-oxyl)-1-methoxylcarbonylethylamino)-phosphonyl]-methoxyl]-propyl]-adenine (I₁₉)

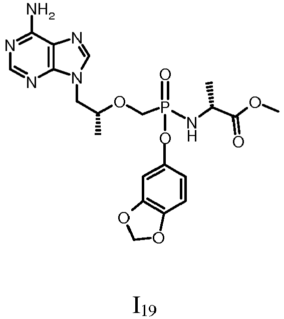

29.2 g of 9-[(R)-2-(phosphonomethoxyl)-propyl]-adenine (PMPA) and 27.6 g of 5-hydroxylbenzo[1,3]dioxolane were added to 150 ml of N-methylpyrrolidone and heated to 85 °C, and 12 ml of triethylamine was added. 26 g of DCC was slowly added with stirring and heated and stirred at 100°C overnight, and 100 ml of water was added after cooling. After standing, the solid was filtered and the filtrate was combined and evaporated under reduced pressure to dryness. 100 ml of water was added, and the pH was adjusted to 11 with 25% sodium hydroxide, the solid was filtered and washed with water, and the washing filtrate was combined and extracted with 30 ml of ethyl acetate. The pH of the aqueous solution was adjusted to 3.1 with 37% hydrochloric acid, the solution was left and the solid was precipitated out. The solid was collected by filtration, and then washed by adding 50 ml of methanol under stirring, filtered and dried under vacuum, to obtain 13.5 g of white solid of 9-[(R)-2-[(((benzo[1,3]dioxolan-5-yl)-oxyl)-phosphonyl]-methoxyl]-propyl]-adenine (III₁).

1.42 g of product III₁ from the last step was added to 10 ml of acetonitrile, 1.3g of sulfoxide chloride was added dropwise with stirring, and the temperature was kept below 50°C. The reaction mixture was heated at 75°C until the solid was dissolved, and then heated to 80 °C and evaporated to dryness, to obtain 9-[(R)-2-[(((benzo[1,3]dioxolan-5-yl)-oxyl)-monochloro-phosphonyl]-methoxyl]-propyl]-adenine (IV₁), which was directly used in the next step reaction.

The IV₁ was dissolved in 10 ml of dry dichloromethane, and cooled to -30°C; a solution of 1.3g of L-alanine methyl ester in 10 ml of dichloromethane was added dropwise over 30 minutes, and the temperature was kept at -18°C. 1.4 ml of triethylamine was then added dropwise over 15 minutes, and the temperature was kept from -18°C to -11°C. It was stirred at room temperature for 2 hours, and washed with 10% sodium dihydrogen phosphate solution (3X10 ml). The organic layer was dried by anhydrous sodium sulfate, and after filtering, the filtrate was evaporated under reduced pressure to dryness, the residue was separated by silica gel column chromatography and eluted with acetone, the desired component was collected and evaporated under reduced pressure to dryness, the residue was separated by silica gel column chromatography, eluted with a mixed solvent of dichloromethane: methanol (95:5), and the desired component to be eluted was collected to obtain 0.41g of I₁₉. H¹-NMR δ (ppm, DMSO-d6): 8.08 (s, 1H); 8.07 (s, 1H); 7.33 (b, 2H); 6.77-6.75 (d, 1H); 6.68 (s, 1H); 6.49-6.47 (d, 1H); 5.99 (s, 2H); 4.41 (m, 1H); 3.92-3.90 (m, 2H); 3.79-3.77 (d, 2H); 3.59 (s, 3H); 3.45 (m, 1H); 1.28 (d, 3H); 1.25 (d, 3H).

### Example 18: Preparation of 9-[(R)-2-[(((benzo[1,3]dioxolan-5-yl)-oxyl)-1-ethoxylcarbonylet hylamino)-phosphonyl]-methoxyl]-propyl]-adenine (I₂₀)

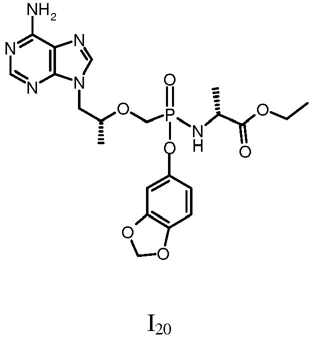

With reference to the method of Example 17, L-alanine ethyl ester was used instead of L-alanine methyl ester to react with IV₁, and the reaction product was washed with 10% sodium dihydrogen phosphate solution (3X10 ml). The organic layer was dried by anhydrous sodium sulfate, and after filtering, the filtrate was evaporated under reduced pressure to dryness, the residue was separated by silica gel column chromatography and eluted with acetone, and the desired component was collected and evaporated under reduced pressure to dryness, to obtain 0.73 g of oily matter, and the oily matter was separated by silica gel column chromatography, eluted with a mixed solvent of dichloromethane: methanol (95:5), and the desired component to be eluted was collected to obtain 0.41g of I₁₉. Upon treatment, separation and purification by similar methods, the title compound I₂₀ was obtained, with a yield of 11.3%. H¹-NMR δ (ppm, DMSO-d6): 8.08 (s, 1H); 8.07 (s, 1H); 7.33 (b, 2H); 6.77-6.75 (d, 1H); 6.68 (s, 1H); 6.49-6.47 (d, 1H) ; 5.99 (s, 2H); 4.41 (m, 1H); 3.92-3.90 (m, 2H); 3.79-3.77 (d, 2H); 3.62 (m, 2H); 3.45 (m, 1H); 1.28 (d, 3H); 1.25 (d, 3H), 1.15 (m, 3H).

### Example 19: Preparation of 9-[(R)-2-[(((benzo[1,3]dioxolan-5-yl)-oxyl)-1-isopropoxycarbonyl ethylamino)-phosphonyl]- methoxyl]-propyl]-adenine (I₂₁) and isomers thereof

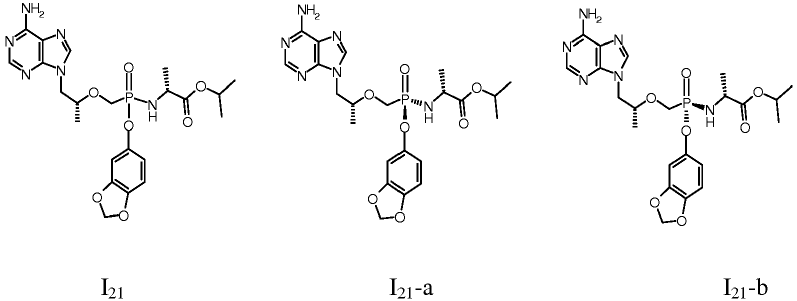

2.84g of III₁ was added to 20 ml of acetonitrile, 2.6 g of sulfoxide chloride was added dropwise with stirring and the temperature was kept below 50°C. The reaction mixture was heated at 75°C until the solid was dissolved, and then heated to 80°C and evaporated to dryness, to obtain IV₁, which was directly used in the next step reaction. The IV₁ was dissolved in 20 ml of dry dichloromethane and cooled to -30°C; a solution of 3.1g of L-alanine methyl ester in 20 ml of dichloromethane was added dropwise over 30 minutes and the temperature was kept at -18°C. 2.8 ml of triethylamine was then added dropwise over 15 minutes, and the temperature was kept from -18°C to -11°C. It was stirred at room temperature for 2 hours and washed with 10% sodium dihydrogen phosphate solution (3X20 ml). The organic layer was dried by anhydrous sodium sulfate, and after filtering, the filtrate was evaporated under reduced pressure to dryness, the residue was separated by silica gel column chromatography and eluted with acetone, the desired component was collected and evaporated under reduced pressure to dryness, the residue was separated by silica gel column chromatography and eluted with a mixed solvent of dichloromethane: isopropanol (95:5), and the desired component to be eluted was collected and evaporated under reduced pressure to dryness to obtain 0.75g of I₂₁.

The I₂₁ was separated by chiral preparative chromatography (Diacel's Chiralpak AS), eluted with acetonitrile containing 30% methanol, and the first main peak was collected and evaporated under reduced pressure to dryness to obtain 0.32g of 9-[(R)-2-[[ (R)-[[(S)-1-(isopropoxycarbonyl)-ethyl]-amino]-(benzo[1,3]dioxolan-5-yl)-oxyl)-phosphonyl]-methoxyl]-propyl]-adenine (I₂₁-a); the second main peak was collected and evaporated under reduced pressure to dryness to obtain 0.35 g of 9-[(R)-2-[[ (S)-[[(S)-1-(isopropoxycarbonyl)-ethyl]-amino]-(benzo[1,3]dioxolan-5-yl)-oxyl)-phosphonyl]-methoxyl]-propyl]-adenine (I₂₁-b).

H¹-NMR of I₂₁-a δ (ppm, DMSO-d6): 8.08 (s, 1H); 8.07 (s, 1H); 7.33 (b, 2H); 6.77-6.75 (d, 1H); 6.68 (s, 1H); 6.49-6.47 (d, 1H); 5.99 (s, 2H); 4.41 (m, 1H); 3.92-3.90 (m, 2H); 3.79-3.77 (d, 2H); 3.76 (m, 1H); 3.45 (m, 1H); 1.27 (d, 3H); 1.20 (d, 3H); 1.15 (d, 6H).

H¹-NMR of I₂₁-b δ (ppm, DMSO-d6): 8.08 (s, 1H); 8.07 (s, 1H); 7.33 (b, 2H); 6.77-6.75 (d, 1H); 6.68 (s, 1H); 6.49-6.47 (d, 1H) ; 5.99 (s, 2H); 4.41 (m, 1H); 3.92-3.90 (m, 2H); 3.79-3.77 (d, 2H); 3.76 (m, 1H); 3.45 (m, 1H); 1.26 (d, 3H); 1.22 (d, 3H); 1.13 (d, 6H).

### Example 20: Preparation of 9-[(R)-2-[(((benzo[1,3]dioxolan-5-yl)-oxyl)-1-isopropoxycarbonyl-2-methyl-propylamino)-phosphonyl]-methoxyl]-propyl]-adenine (I₂₂)

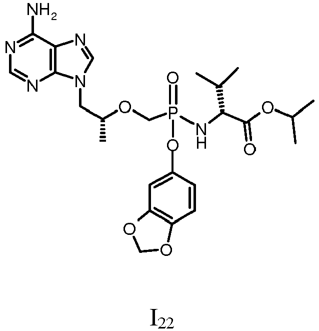

With reference to the method of Example 17, L-valine isopropyl ester was used instead of L-alanine methyl ester to react with IV₁, and after treatment, separation and purification by similar methods, the title compound I₂₂ was obtained, with a yield of 18.2%. H¹-NMR δ (ppm, DMSO-d6): 8.08 (s, 1H); 8.07 (s, 1H); 7.33 (b, 2H); 6.77-6.75 (d, 1H); 6.68 (s, 1H); 6.49-6.47 (d, 1H); 5.99 (s, 2H); 4.41 (m, 1H); 3.92-3.90 (m, 2H); 3.79-3.77 (d, 2H); 3.76 (m, 1H); 3.45 (m, 1H); 1.09-1.28 (m, 16H).

### Example 21: Preparation of 9-[(R)-2-[(((benzo[1,3]dioxolan-5-yl)-oxyl)-1-isopropoxycarbonyl-3-methyl-butylamino)- phosphonyl]-methoxyl]-propyl]-adenine (I₂₃)

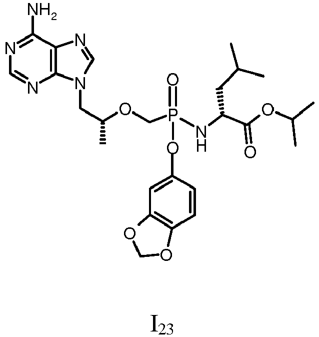

With reference to the method of Example 17, L-leucine isopropyl ester was used instead of L-alanine methyl ester to react with IV₁, and after treatment, separation and purification by similar methods, the title compound I₂₃ was obtained, with a yield of 12.8%. H¹-NMR δ (ppm, DMSO-d6): 8.08 (s, 1H); 8.07 (s, 1H); 7.33 (b, 2H); 6.77-6.75 (d, 1H); 6.68 (s, 1H); 6.49-6.47 (d, 1H); 5.99 (s, 2H); 4.41 (m, 1H); 3.92-3.90 (m, 2H); 3.79-3.77 (d, 2H); 3.76 (m, 1H); 3.45 (m, 1H); 1.09-1.28 (m, 18H).

### Example 22: Preparation of 9-[(R)-2-[(((benzo[1,3]dioxolan-4-yl)-oxyl)-1-isopropoxycarbonyl ethylamino)-phosphonyl]- methoxyl]-propyl]-adenine (I₂₄)

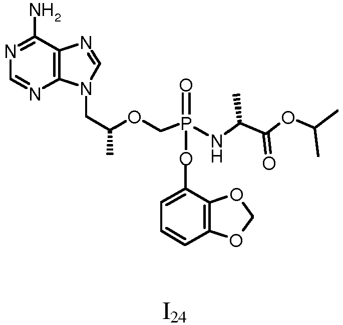

With reference to the method of Example 17, 4-hydroxylbenzo[1,3]dioxolane was used instead of 5-hydroxylbenzo[1,3]dioxolane to react with PMPA, and after treatment, separation and purification by similar methods, 9-[(R)-2-[(((benzo[1,3]dioxolan-4-yl)-oxyl)-phosphonyl]-methoxyl]-propyl]-adenine(III₂) was obtained.

With reference to the method of Example 17, the III₂ was used instead of III₁ to react with sulfoxide chloride, and after treatment, separation and purification by similar methods, 9-[(R)-2-[(((benzo[1,3]dioxolan-4-yl)-oxyl)-monochloro-phosphonyl]-methoxyl]-propyl]-adenine (IV₂) was obtained.

With reference to the method of Example 17, the IV₂ was used instead of IV₁ to react with L-alanine isopropyl ester, and after treatment, separation and purification by similar methods, I₂₄ was obtained, with a yield of 13.7%, H¹-NMR δ (ppm, DMSO-d6): 8.08 (s, 1H); 8.07 (s, 1H); 7.33 (b, 2H); 6.67-6.71 (t, 1H); 6.58-6.60 (d, 1H); 6.53-6.55 (d, 1H); 5.97 (s, 2H); 4.41 (m, 1H); 3.92-3.90 (m, 2H); 3.79-3.77 (d, 2H); 3.63 (m, 1H); 3.45 (m, 1H); 1.12-1.28 (m, 12H).

### Example 23: Preparation of 9-[(R)-2-[(((benzo[1,3]dioxolan-5-yl)-oxyl)-1-cyclohexyloxy carbonylethylamino)-phosphonyl]-methoxyl]-propyl]-adenine (I₂₅)

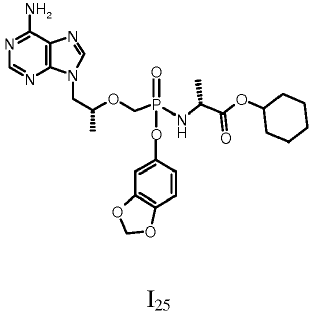

With reference to the method of Example 17, L-alanine cyclohexyl ester was used instead of L-alanine methyl ester to react with IV₁, and after treatment, separation and purification by similar methods, the title compound I₂₅ was obtained, with a yield of 16.3%. H¹-NMR δ (ppm, DMSO-d6): 8.08 (s, 1H); 8.07 (s, 1H); 7.33 (b, 2H); 6.77-6.75 (d, 1H); 6.68 (s, 1H); 6.49-6.47 (d, 1H); 5.99 (s, 2H); 4.41 (m, 1H); 3.92-3.90 (m, 2H); 3.79-3.77 (d, 2H); 3.64 (m, 1H); 3.45(m, 1H); 1.58 (m, 4H), 1.18-1.39 (m, 9H).

### Example 24: Preparation of 2-amino-6-(4-methoxylphenyl)thio-9-[[[(((benzo[1,3]dioxolan-5-yl)-oxyl)-1-isopropyloxylcarbonylethylamino)-phosphonyl]-methoxyl]-ethyl]-purine (I₂₆)

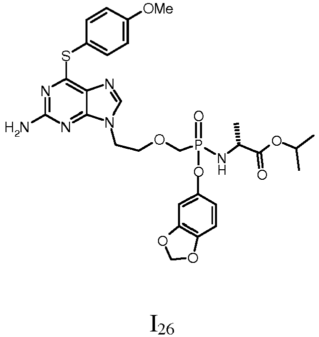

With reference to the method of Example 17, 2-amino-6-(4-methoxylphenyl)thio-9-[(phosphonyl-methoxyl)-ethyl]-purine was used instead of PMPA to react with 5-hydroxylbenzo[1,3]dioxolane, and after treatment, separation and purification by similar methods, 2-amino-6-(4-methoxylphenyl)thio-9-[[[(((benzo[1,3]dioxolan-5-yl)-oxyl)-phosphonyl]-methoxyl]-ethyl]-purine (III₃) was obtained.

With reference to the method of Example 17, the III₃ was used instead of III₁ to react with sulfoxide chloride, and after treatment, separation and purification by similar methods, 2-amino-6-(4-methoxylphenyl)thio-9-[[[(((benzo[1,3]dioxolan-5-yl)-oxyl)-monochlorophospho nyl]-methoxyl]-ethyl]-purine(IV₃) was obtained.

With reference to the method of Example 17, the IV₃ was used instead of IV₁ to react with L-alanine isopropyl ester, and after treatment, separation and purification by similar methods, I₂₆ was obtained, with a yield of 10.4%, H¹-NMR δ (ppm, DMSO-d6): 7.89 (s,1H); 7.54 (d,2H); 7.32 (b, 2H); 6.91 (d,2H); 6.77-6.75 (d, 1H); 6.68 (s, 1H); 6.49-6.47 (d, 1H); 5.99 (s, 2H); 4.35-4.33 (t, 2H); 3.93-3.91 (t, 2H); 3.89 (s, 3H); 3.80-3.78 (d, 2H); 3.63 (m, 1H); 3.45 (m, 1H); 1.12-1.28 (m, 9H).

### Example 25: Preparation of 9-[[[(((benzo[1,3]dioxolan-5-yl)-oxyl)-1-isopropyloxylcarbonyl ethylamino)-phosphoryl]-oxyl]-ethoxyl]-methyl]-guanine (I₂₇)

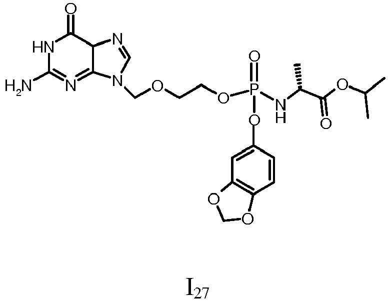

15.3 g of phosphorus oxychloride and 13.8g of 5-hydroxylbenzodioxolane were added to 250 ml of anhydrous diethyl ether solution, and cooled to -78°C under the protection of Ar, and 13.4 ml of triethylamine was added dropwise, and after adding, the solution was stirred for 30 minutes at -78°C and then stirred at room temperature overnight. Filtered, and the filtrate was evaporated under reduced pressure to dryness, to obtain benzo[1,3]dioxolan-5-yl)-oxyl-phosphorus oxydichloride for later use.

2.6 g of (0.01 mol) benzo[1,3]dioxolan-5-yl)-oxyl-phosphorus oxydichloride and 1.6 g of (0.01 mol) L-alanine isopropyl ester were dissolved in 30 ml of anhydrous dichloromethane and cooled to -78°C. A solution of 2 ml of triethylamine dissolved in 20 ml of anhydrous dichloromethane was added dropwise with stirring, and the rate of dropwise adding was controlled to keep the reaction tempetature at -78°C. After adding, when the reaction temperature was slowly raised to room temperature, stirring was continued for 1 hour. The solvent was evaporated under reduced pressure, and 30 ml of anhydrous diethyl ether was added to the residue, and then filtered. The filtrate was evaporated under reduced pressure to dryness so as to obtain a colourless oily matter, i.e., phosphoramide intermediate V₃, which was directly used in the next step reaction.

0.22 g of acyclovir was dissolved in 50 ml of THF, and then 7 ml of pyridine was added, purged with nitrogen, 1 ml of 1M solution of tert-butyl magnesium chloride in THF was added and stirred for 0.5 h, and 2 ml of 1M solution of V₃ in THF was added with stirring and stirred for 2 hours. 50 ml of dichloromethane was added to the reaction solution, after being extracted with 100 ml of saturated aqueous solution of ammonium chloride, the organic layer was separated, and the aqueous layer was extracted with dichloromethane (3X50ml), the organic layer was combined, washed with a saturated aqueous solution of sodium chloride, and then dried by anhydrous magnesium sulfate overnight and filtered, the filtrate was evaporated under reduced pressure to dryness, the residue was separated by silica gel column chromatography, eluted with a mixed solvent of dichloromethane: methanol: triethylamine (100:5:1), and the desired component was collected and evaporated to dryness to obtain 0.15g of I₂₇. H¹-NMR (ppm, DMSO-d6): 10.65 (s, 1H); 7.81 (s, 1H); 6.77-6.75 (d, 1H); 6.68 (s, 1H); 6.5 (bs, 2H); 6.49-6.47 (d, 1H); 5.99 (s, 2H); 5.35 (s, 2H); 4.1-4.0 (t, 2H); 3.7-3.6 (m, 3H); 3.45 (m, 1H); 1.12-1.28 (m, 9H).

### Example 26: Preparation of 9-[[[(((benzo[1,3]dioxolan-5-yl)-oxyl)-1-cyclohexyloxylcarbonyl ethylamino)-phosphoryl]-oxyl]-ethoxyl]-methyl]-guanine (I₂₈)

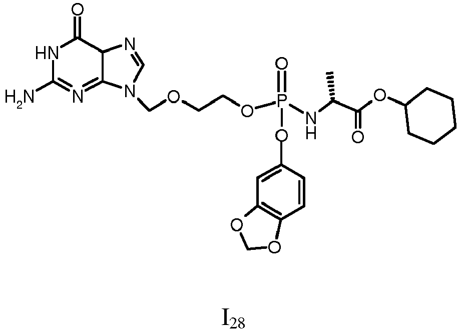

With reference to the method of Example 25, L-alanine cyclohexyl ester was used instead of L-alanine isopropyl ester to react with benzo[1,3]dioxolan-5-yl)-oxyl-phosphorus oxydichloride, and after treatment, separation and purification by similar methods, phosphoramide intermediate V₄ was obtained, directly for use in the next step reaction.

The V₄ was used instead of V₃ to react with acyclovir, and after treatment, separation and purification by similar methods, the title compound I₂₈ was obtained, with a yield of 14.2%. H¹-NMR (ppm, DMSO-d6): 10.65 (s, 1H); 7.81 (s, 1H); 6.77-6.75 (d, 1H); 6.68 (s, 1H); 6.5 (bs, 2H); 6.49-6.47 (d, 1H); 5.99 (s, 2H); 5.35 (s, 2H); 4.1-4.0 (t, 2H); 3.7-3.6 (m, 3H); 3.45 (m, 1H); 1.55 (m, 4H), 1.15-1.37 (m, 9H).

### Example 27: Preparation of 2-ammo-6-methoxyl-9-[[[(((benzo[1,3]dioxolan-5-yl)-oxyl)-1-isopropyloxylcarbonylethylamino)-phosphoryl]-oxyl]-ethoxyl]-methyl]-purine (I₂₉)

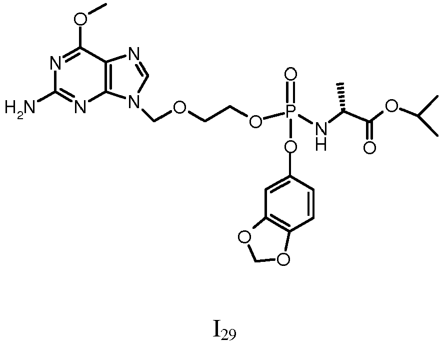

With reference to the method of Example 25, 2-amino-6-methoxyl-9-[(2-hydroxylethoxyl)-methyl]-purine was used instead of acyclovir to react with V₃, and after treatment, separation and purification by similar methods, the title compound I₂₉ was obtained, with a yield of 16.9%. H¹-NMR (ppm, DMSO-d6): 7.98 (s, 1H); 6.77-6.75 (d, 1H); 6.68 (s, 1H); 6.5 (bs, 2H); 6.49-6.47 (d, 1H); 5.99 (s, 2H); 5.35 (s, 2H); 3.91 (s, 3H); 4.1-4.0 (t, 2H); 3.7-3.6 (m, 3H); 3.45 (m, 1H); 1.12-1.28 (m, 9H).

### Example 28: Preparation of 5'-[((((benzo[1,3]dioxolan-5-yl)-oxyl)-1-ethoxylcarbonyl ethylamino)-phosphoryl]-β-D-2'-deoxy-2'-α-fluoro-2'-β-C-methyluridine (I₃₀)

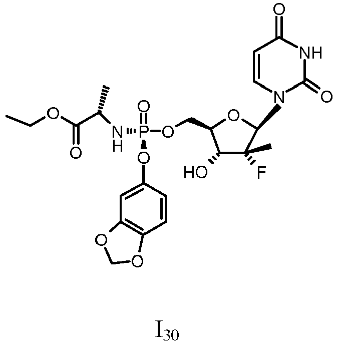

With reference to the method of Example 25, L-alanine ethyl ester was used instead of L-alanine isopropyl ester to react with benzo[1,3]dioxolan-5-yl)-oxyl-phosphorus oxydichloride, and after treatment, separation and purification by similar methods, phosphoramide intermediate V₅ was obtained, directly for use in the next step reaction.

With reference to the method of Example 25, the V₅ was used to react with β-D-2'-deoxy-2'-α-fluoro-2'-β-C-methyluridine, and after treatment, separation and purification by similar methods, the title compound I₃₀ was obtained, with a yield of 13.5%. H¹-NMR (ppm, DMSO-d6): 11.37 (s, 1H); 7.82 (d, 1H); 6.77-6.75 (d, 1H); 6.68 (s, 1H); 6.49-6.47 (d, 1H); 5.99 (s, 2H); 5.95 (d, 1H); 5.51 (d, 1H); 3.82-3.70 (m, 3H); 3.63-3.60 (m, 3H); 3.45 (m, 1H); 1.12-1.28 (m, 9H).

### Example 29: Preparation of 5'-[((((benzo[1,3]dioxolan-5-yl)-oxyl)-1-isopropoxycarbonylethyl amino)-phosphoryl]-β-D-2'-deoxy-2'-α-fluoro-2'-β-C-methyluridine (I₃₁) and isomers thereof

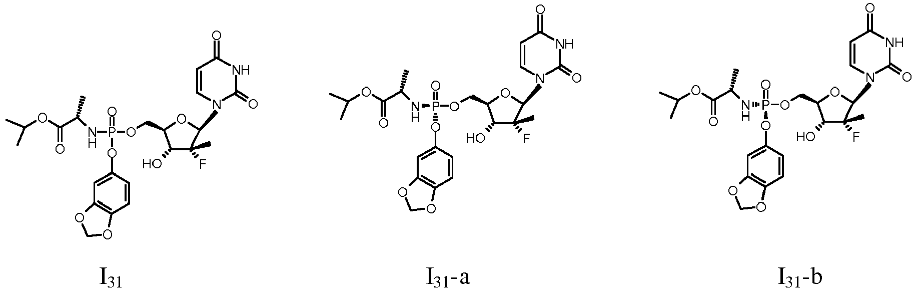

4.5g of β-D-2'-deoxy-2'-α-fluoro-2'-β-C-methyluridine (Ji'nan Branch of A Chemical Co., Ltd., the purity of 98%) was dissolved in 80 ml of THF, and then 9g of N-methylimidazole was added, a solution of 10.5 g of V₃ dissolved in 50 ml of THF was added. It was Stirred at room temperature overnight, filtered, and the filtrate was evaporated under reduced pressure to dryness. The residue was separated by silica gel column chromatography, eluted with dichloromethane containing 2% isopropanol, and the desired component was collected and evaporated under reduced pressure to dryness. The residue was dissolved with acetonitrile containing 20% isopropanol and separated by chiral preparative chromatography, with the chromatography column of Diacel's Chiralpak AS, the mobile phase being acetonitrile solution containing 20% isopropanol, the flow rate being 8 ml/min, and the first main peak was collected and evaporated under reduced pressure to dryness to obtain 0.28g of I₃₁-a; the second main peak was collected and evaporated under reduced pressure to dryness to obtain 0.23g of I₃₁-b.

H¹-NMR of I₃₁-a (ppm, DMSO-d6): 11.37 (s, 1H); 7.82 (d, 1H); 6.77-6.75 (d, 1H); 6.68 (s, 1H); 6.49-6.47 (d, 1H); 5.99 (s, 2H); 5.95 (d, 1H); 5.51 (d, 1H); 3.82-3.70 (m, 3H); 3.63-3.60 (m, 2H); 3.45 (m, 1H); 1.25 (d, 3H); 1.20 (d, 3H); 1.15 (d, 6H).

H¹-NMR of I₃₁-b (ppm, DMSO-d6): 11.37 (s, 1H); 7.82 (d, 1H); 6.77-6.75 (d, 1H); 6.68 (s, 1H); 6.49-6.47 (d, 1H); 5.99 (s, 2H); 5.95 (d, 1H); 5.51 (d, 1H); 3.82-3.70 (m, 3H); 3.63-3.60 (m, 2H); 3.45 (m, 1H); 1.24 (d, 3H); 1.21 (d, 3H); 1.14 (d, 6H).

### Example 30: Preparation of 5'-[((((benzo[1,3]dioxolan-5-yl)-oxyl)-1-cyclohexyloxylcarbonyl ethylamino)-phosphoryl]-β-D-2'-deoxy-2'-α-fluoro-2'-β-C-methyluridine (I₃₂)

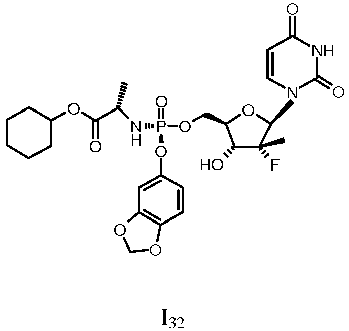

With reference to the method of Example 25, 2-amino-6-methoxyl-9-[(1S,3R,4S)-4-hydroxyl-3-hydroxymethyl)-2-methylenecyclopentyl]-purine was reacted with V₄, and after treatment, separation and purification by similar methods, the title compound I₃₂ was obtained, with the yield of 9.8%. H¹-NMR (ppm, DMSO-d6): 11.37 (s, 1H); 7.82 (d, 1H); 6.77-6.75 (d, 1H); 6.68 (s, 1H); 6.49-6.47 (d, 1H); 5.99 (s, 2H); 5.95 (d, 1H); 5.51 (d, 1H); 3.82-3.70 (m, 3H); 3.63-3.60 (m, 2H); 3.45 (m, 1H); 1.55 (m, 4H), 1.15-1.37 (m, 12H).

### Example 31: Preparation of 5'-[((((benzo[1,3]dioxolan-5-yl)-oxyl)-1-ethoxylcarbonylethyl amino)-phosphoryl]-β-D-2'-deoxy-2'-α-fluoro-2'-β-C-methyl-6-O-methylguanosine (I₃₃)

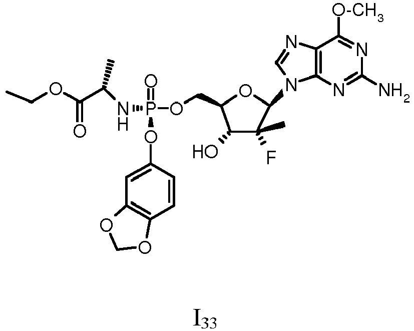

With reference to the method of Example 25, V₅ was used to react with β-D-2'-deoxy-2'-α-fluoro-2'-α-C-methyl-6-O-methylguanosine, and after treatment, separation and purification by similar methods, the title compound I₃₃ was obtained, with the yield of 24.2%. H¹-NMR (ppm, DMSO-d6): 7.98 (s, 1H); 6.77-6.75 (d, 1H); 6.68 (s, 1H); 6.52 (s, 2H); 6.49-6.47 (d, 1H); 6.08-5.92 (m, 4H); 3.91 (s, 3H); 3.85-3.73 (m, 2H); 3.62 (m, 2H); 3.45 (m, 1H); 1.25 (d, 3H); 1.21 (d, 3H), 1.15 (m, 3H).

### Example 32: Preparation of 5'-[((((benzo[1,3]dioxolane-5-yl)-oxyl)-1-isopropoxycarbonylethylamino)-phosphoryl]-β-D-2'-deoxy-2'-α-fluoro-2'-β-C-methyl-6-O-methylguanosine (I₃₄) and isomers thereof

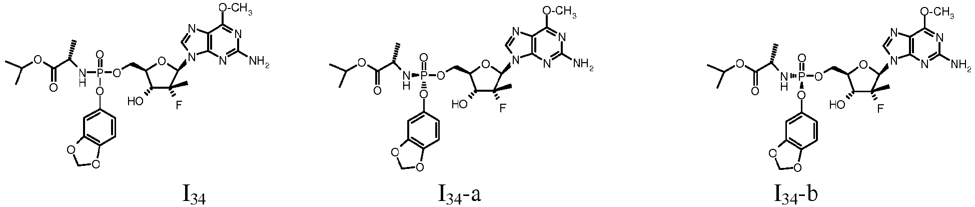

6.0 g of β-D-2'-deoxy-2'-α-fluoro-2'-β-C-methyl-6-O-methylguanosine (Ji'nan Branch of A Chemical Co., Ltd., the purity of 98%) was dissolved in 80ml of THF, and then 9 g of N-methylimidazole was added, a solution of 10.5 g of V₃ dissolved in 50 ml of THF was added. It was stirred at room temperature overnight, filtered, and the filtrate was evaporated under reduced pressure to dryness. The residue was separated by silica gel column chromatography, eluted with dichloromethane containing 2% isopropanol, the desired component was collected and evaporated under reduced pressure to dryness. The residue was dissolved with acetonitrile containing 20% isopropanol and separated by chiral preparative chromatography, with the chromatography column of Diacel's Chiralpak AS, the mobile phase being acetonitrile solution containing 20% isopropanol, the flow rate being 8 ml/min, the first main peak was collected and evaporated under reduced pressure to dryness to obtain 0.31 g of I₃₄-a; the second main peak was collected and evaporated under reduced pressure to dryness to obtain 0.25 g of I₃₄-b.

H¹-NMR of I₃₄-a (ppm, DMSO-d6): 7.98(s, 1H); 6.77-6.75 (d, 1H); 6.68 (s, 1H); 6.52 (s, 2H); 6.49-6.47 (d, 1H); 6.08-5.92 (m, 4H); 3.91 (s, 3H); 3.85-3.73 (m, 3H); 3.45 (m, 1H); 1.25 (d, 3H); 1.22 (d,3H); 1.14 (d, 6H).

H¹-NMR of I₃₄-b (ppm, DMSO-d6): 7.98(s, 1H); 6.77-6.75 (d, 1H); 6.68 (s, 1H); 6.52 (s, 2H); 6.49-6.47 (d, 1H); 6.08-5.92 (m, 4H); 3.91 (s, 3H); 3.85-3.73 (m, 3H); 3.45 (m, 1H); 1.24 (d, 3H); 1.21 (d,3H); 1.13 (d, 6H).

### Example 33: Preparation of 5'-[((((benzo[1,3]dioxolan-5-yl)-oxyl)-1-cyclohexyloxycarbonyle thylamino)-phosphoryl]-β-D-2'-deoxy-2'-α-fluoro-2'-β-C-methyl-6-O-methylguanosine (I₃₅)

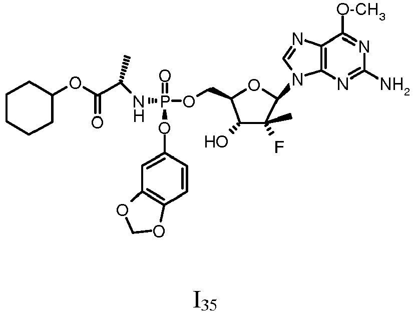

With reference to the method of Example 25, 5'-O-phosphoryl-β-D-2'-deoxy-2'-α-fluoro-2'-β-C-methyl-6-O-methylguanosine was reacted with V₄, and after treatment, separation and purification by similar methods, the title compound I₃₅ was obtained, with a yield of 13.0%. H¹-NMR (ppm, DMSO-d6): 7.98 (s, 1H); 6.77-6.75 (d, 1H); 6.68 (s, 1H); 6.52 (s, 2H); 6.49-6.47 (d, 1H); 6.08-5.92 (m, 4H); 3.91 (s, 3H); 3.85-3.73 (m, 3H); 3.45 (m, 1H); 1.55 (m, 4H); 1.15-1.37 (m, 12H).

### Example 34: Preparation of 5'-[((((benzo[1,3]dioxolan-5-yl)-oxyl)-1-isopropoxycarbonyl ethylamino)-phosphoryl]-2',3'-dideoxy-3'-thia-cytidine (I₃₆)

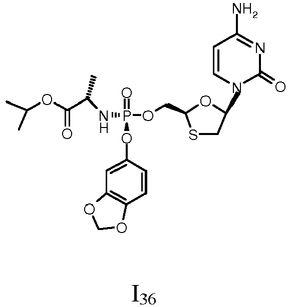

With reference to the method of Example 25, lamivudine was used instead of acyclovir to react with V₃, and after treatment, separation and purification by similar methods, the title compound I₃₆ was obtained. H¹-NMR δ (ppm, DMSO-d6): 7.68 (d, 1H); 7.26 (s, 2H); 6.77-6.75 (d, 1H); 6.68 (s,1H); 6.49-6.47 (d, 1H); 6.23 (t, 1H); 6.11 (m, 1H); 5.99 (s, 2H); 5.72 (d,1H); 5.35 (t,1 H); 4.27 (m, 2H); 3.88 (m, 1H); 3.61 (m,1H); 3.42 (q , 1H); 3.05 (m, 2H); 1.21 (d, 3H); 1.12 (d, 6H).

### Example 35: Preparation of 5'-[((((benzo[1,3]dioxolan-5-yl)-oxyl)-1-methoxylcarbonylethyla mino)-phosphoryl]-2',3'-dideoxy-3'-thia-cytidine (I₃₇)

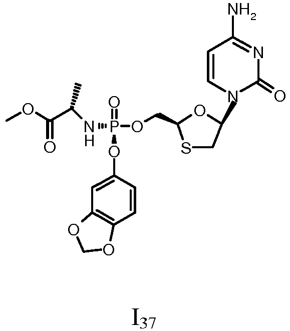

With reference to the method of Example 25, L-alanine methyl ester was used instead of L-alanine isopropyl ester to react with benzo[1,3]dioxolan-5-yl)-oxyl-phosphorus oxydichloride, and after treatment, separation and purification by similar methods, phosphoramide intermediate V₆ was obtained, directly for use in the next step reaction.

With reference to the method of Example 25, the V₆ was used to react with lamivudine, and after treatment, separation and purification by similar methods, the title compound I₃₇ was obtained, with a yield of 21.7%. H¹-NMR δ (ppm, DMSO-d6): 7.68 (d, 1H); 7.26 (s, 2H); 6.77-6.75 (d, 1H); 6.68 (s,1H); 6.49-6.47 (d, 1H); 6.23 (t, 1H); 6.11 (m, 1H); 5.99 (s, 2H); 5.72 (d,1H); 5.35 (t ,1 H); 4.27 (m, 2H); 3.88 (m, 1H); 3.58 (s ,3H); 3.42 (q, 1H); 3.05 (m, 2H); 1.21 (d, 3H).

### Example 36: Preparation of 9-[(1S,3R,4S)-4-hydroxyl-3-[(((benzo[1,3]dioxolan-5-yl)-oxyl)-1-isopropoxycarbonylethylamino-phosphoryl)-oxymethyl]-2-methylenecyclopentyl]-guanine (I₃₈) and the isomers thereof

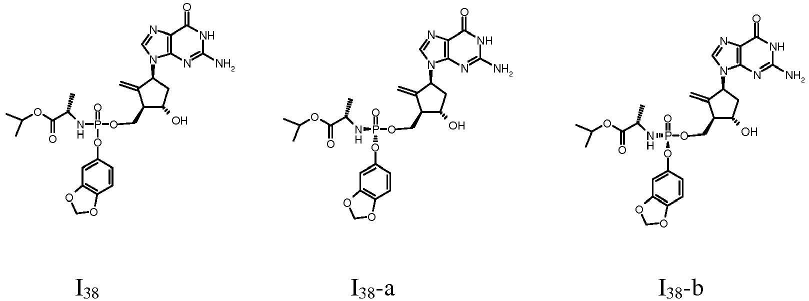

8.25 g of entecavir was dissolved in 80 ml of THF, and then 9 g of N-methylimidazole was added, a solution of 10.5 g of V₃ dissolved in 50 ml of was added. It was stirred at room temperature overnight, filtered, and the filtrate was evaporated under reduced pressure to dryness. The residue was separated by silica gel column chromatography, eluted with dichloromethane containing 2% isopropanol, the desired component was collected and evaporated under reduced pressure to dryness. The residue was dissolved with acetonitrile containing 20% isopropanol and separated by chiral preparative chromatography, with the chromatography column of Diacel's Chiralpak AS, the mobile phase being acetonitrile solution containing 20% isopropanol, the flow rate being 8 ml/min, the first main peak was collected and evaporated under reduced pressure to dryness to obtain 0.28 g of I₃₉-a; the second main peak was collected and evaporated under reduced pressure to dryness to obtain 0.22 g of I₃₉-b.

H¹-NMR of I₃₉-a δ (ppm, DMSO-d6, 400MHz): 10.51(bs, 1H); 7.63 (s, 1H); 6.77-6.75 (d, 1H); 6.68 (s,1H); 6.49-6.47 (d, 1H); 6.09 (m, 1H); 6.45 (s, 2H); 5.99 (s, 2H); 5.32 (m, 1H); 5.06 (m, 1H); 4.89 (d, 1H); 4.85-4.83 (m, 2H); 4.52 (m, 1H); 4.40 (m, 1H); 4.23 (m, 1H); 3.51 (m, 2H); 2.52 (m, 1H); 2.22 (m, 1H); 2.04 (m, 1H); 1.19 (d, 3H); 1.16 (d , 6H).

H¹-NMR of I₃₉-b δ (ppm, DMSO-d6, 400MHz): 10.54 (bs, 1H); 7.67 (s, 1H); 6.77-6.75 (d, 1H); 6.68 (s,1H); 6.49-6.47 (d, 1H); 6.45 (s, 2H); 5.98-6.03 (m, 3H); 5.80 (m, 1H); 5.32 (m, 1H); 5.07 (m, 1H); 4.87-4.83 (m, 3H); 4.58 (m,1H); 4.35 (m, 1H); 4.23 (m, 1H); 3.51 (m, 2H); 2.50 (m,1H); 2.21 (m, 1H); 2.03 (m, 1H); 1.21 (d, 3H); 1.13 (d, 6H).

### Example 37: Preparation of 9-[(1S,3R,4S)-4-hydroxyl-3-[(((benzo[1,3]dioxolan-4-yl)-oxyl)-1-isopropoxycarbonylethylamino-phosphoryl)-oxymethyl]-2-methylenecyclopentyl]-guanine (I₃₉)

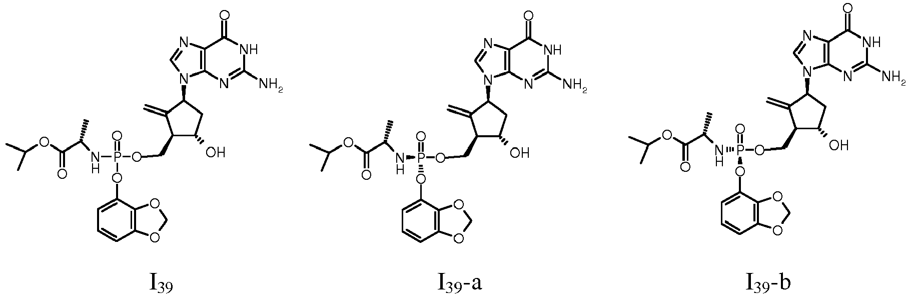

15.3 g of phosphorus oxychloride and 13.8 g of 4-hydroxylbenzodioxolane were added to a 250 ml of anhydrous diethyl ether solution, and was cooled to -78°C under the protection of Ar, 13.4 ml of triethylamine was added dropwise, and after adding, it was stirred for 30 minutes at -78 °C, and then, stirred at room temperature overnight. Filtered, the filtrate was evaporated under reduced pressure to dryness, to obtain benzo[1,3]dioxolan-4-yl)-oxyl-phosphorus oxydichloride for later use.

2.6 g of (0.01 mol) benzo[1,3]dioxolan-4-yl)-oxyl-phosphorus oxydichloride and 1.6 g of (0.01 mol) L-alanine isopropyl ester were dissolved in 30 ml of anhydrous dichloromethane and cooled to -78°C. A solution of 2 ml of triethylamine dissolved in 20 ml of anhydrous dichloromethane was added dropwise with stirring, and the rate of dropwise adding was controlled to keep the reaction tempetature at -78°C. After adding, when the reaction temperature was slowly raised to room temperature, stirring was continued for 1 hour. The solvent was evaporated under reduced pressure, and 30 ml of anhydrous diethyl ether was added to the residue, and then filtered. The filtrate was evaporated under reduced pressure to dryness so as to obtain a colourless oily matter, i.e., phosphoramide intermediate V₇, which was directly used in the next step reaction.

8.25 g of entecavir was dissolved in 80 ml of THF, and then 9 g of N-methylimidazole was added, and a solution of 10.5 g of V₇ dissolved in 50 ml of THF was added. It was stirred at room temperature overnight, filtered, and the filtrate was evaporated under reduced pressure to dryness. The residue was separated by silica gel column chromatography, eluted with dichloromethane containing 2% isopropanol and the desired component was collected and evaporated under reduced pressure to dryness. The residue was dissolved with acetonitrile containing 20% isopropanol and separated by chiral preparative chromatography, with the chromatography column of Diacel' s Chiralpak AS, the mobile phase being acetonitrile solution containing 20 % isopropanol, the flow rate being 8 ml/min, the first main peak was collected and evaporated under reduced pressure to dryness to obtain 0.27 g of I₃₉-a; the second main peak was collected and evaporated under reduced pressure to dryness to obtain 0.20 g of I₃₉-b.

H¹-NMR of I₃₉-a δ (ppm, DMSO-d6, 400MHz): 10.51 (bs, 1H); 7.63 (s, 1H); 6.67-6.71 (t, 1H); 6.58-6.60 (d, 1H); 6.53-6.55 (d, 1H); 6.09 (m, 1H); 6.45 (s, 2H); 5.99 (s, 2H); 5.32 (m, 1H); 5.06 (m, 1H); 4.89 (d, 1H); 4.85-4.83 (m, 2H); 4.52 (m, 1H); 4.40 (m, 1H); 4.23 (m, 1H); 3.51 (m, 2H); 2.52 (m, 1H); 2.22 (m, 1H); 2.04 (m, 1H); 1.19 (d, 3H); 1.16 (d , 6H).

H¹-NMR of I₃₉-b δ (ppm, DMSO-d6, 400MHz): 10.54 (bs, 1H); 7.67 (s, 1H); 6.67-6.71 (t, 1H); 6.58-6.60 (d, 1H); 6.53-6.55 (d, 1H); 6.45 (s, 2H); 5.98-6.03 (m, 3H); 5.80 (m, 1H); 5.32 (m, 1H); 5.07 (m, 1H); 4.87-4.83 (m, 3H); 4.58 (m ,1H); 4.35 (m, 1H); 4.23 (m, 1H); 3.51 (m, 2H); 2.50 (m,1H); 2.21 (m, 1H); 2.03 (m, 1H); 1.21 (d, 3H); 1.13(d, 6H).

### Example 38: Preparation of 5'-[((((benzo[1,3]dioxolan-4-yl)-oxyl)-1-isopropoxycarbonylethyl amino)-phosphoryl]-β-D-2'-deoxy-2'-α-fluoro-2'-β-C-methyl-6-O-methylguanosine (I₄₀) and isomers thereof

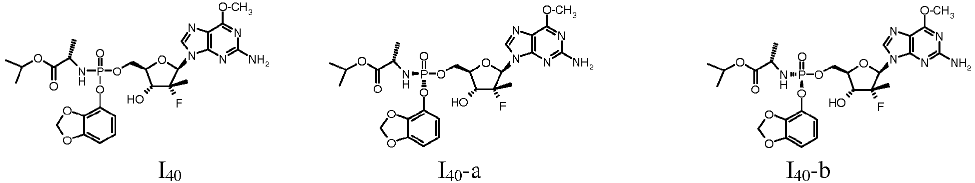

6.0 g of β-D-2'-deoxy-2'-α-fluoro-2'-β-C-methyl-6-O-methylguanosine (Ji'nan Branch of A Chemical Co., Ltd., the purity of 98%) was dissolved in 80 ml of THF, and then 9 g of N-methylimidazole was added and a solution of 10.5g of V₇ dissolved in 50 ml of THF was added. It was stirred at room temperature overnight, filtered, and the filtrate was evaporated under reduced pressure to dryness. The residue was separated by silica gel column chromatography, eluted with dichloromethane containing 2% isopropanol and the desired component was collected and evaporated under reduced pressure to dryness. The residue was dissolved with acetonitrile containing 20% isopropanol and separated by chiral preparative chromatography, with the chromatography column of Diacel's Chiralpak AS, the mobile phase being acetonitrile solution containing 20 % isopropanol, the flow rate being 8 ml/min, the first main peak was collected and evaporated under reduced pressure to dryness to obtain 0.22 g of I₄₀-a; the second main peak was collected and evaporated under reduced pressure to dryness to obtain 0.24 g of I₄₀-b.

H¹-NMR of I₄₀-a (ppm, DMSO-d6): 7.98 (s, 1H); 6.67-6.71 (t, 1H); 6.58-6.60 (d, 1H); 6.53-6.55(d, 1H); 6.52 (s, 2H); 6.08-5.92 (m, 4H); 3.91 (s, 3H); 3.85-3.73 (m, 3H); 3.45 (m, 1H); 1.25 (d, 3H); 1.22 (d,3H); 1.14 (d, 6H).

H¹-NMR of I₄₀-b (ppm, DMSO-d6): 7.98 (s, 1H); 6.67-6.71 (t, 1H); 6.58-6.60 (d, 1H); 6.52-6.55 (m, 3H); 6.08-5.92 (m, 4H); 3.91 (s, 3H); 3.85-3.73 (m, 3H); 3.45 (m, 1H); 1.25 (d, 3H); 1.20 (d,3H); 1.13 (d, 6H).

### Example 39: Preparation of 9-[[[(((benzo[1,3]dioxolan-5-yl)-oxyl)-1-methoxylcarbonylethyl amino)-phosphoryl]-oxyl]- ethoxyl]-methyl]-guanine (I₄₁)

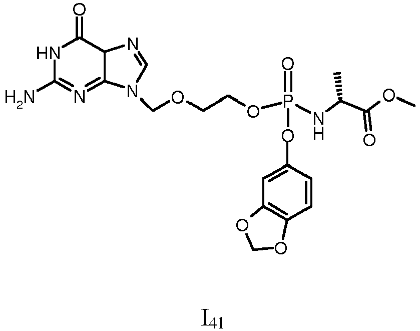

With reference to the method of Example 25, V₆ was used instead of V₃ to react with acyclovir, and after treatment, separation and purification by similar methods, the title compound I₄₁ was obtained, with a yield of 23.3%. 7.83 (s, 1H); 6.77-6.75 (d, 1H); 6.68 (s, 1H); 6.5 (bs, 2H); 6.49-6.47 (d, 1H); 6.00-5.93 (m, 3H); 5.36 (s, 2H); 4.13-4.05 (m, 2H); 3.85-3.80 (m, 1H); 3.72-3.63 (m, 2H); 3.59 (s, 3H); 1.23-1.19 (m, 3H).

### Example 40: Screening of anti-HBV activity in vitro

Hep G 2.2.15 cells were inoculated in a 96-well plate to cultivate, with the cell number being 3.5 X 10⁴, and placed in a CO₂ incubator to incubate until the cell density achieved 80%, the culture solution was discarded, and new culture solutions containing different concentrations of drugs to be tested were added, 3 wells were provided in parallel; and the culture solutions were replaced every other two days. At day 10 post-dosing, 100 µl supernate was taken and the content of HBV DNA was measured by the method of Quantitative PCR to calculate IC₅₀ value. The results are shown in Table 1.

**Table 1 Screening results of anti-HBV activity in vitro**

| Compound | IC₅₀ (µM) | Compound | IC₅₀ (µM) |
|---|---|---|---|
| Lamivudine | 0.075 | I₂₁-a | 0.15 |
| Acyclovir | 25.6 | I₂₁-b | 0.012 |
| i-1 | 11.3 | I₂₂ | 0.17 |
| i-2 | 1.92 | I₂₄ | 4.6 |
| I₁ | 8.05 | I₂₆ | 0.039 |
| I₂ | 0.47 | I₂₇ | 0.035 |
| I₃ | 6.07 | I₂₉ | 0.14 |
| I₄ | 3.19 | i-4 | 0.005 |
| I₆ | 0.41 | i-5 | 0.012 |
| I₈ | 0.93 | I₃₈-a | 0.001 |
| I₁₁ | 1.66 | I₃₈-b | 0.090 |
| I₁₂ | 0.24 | I₃₉-a | 0.017 |
| I₁₃ | 0.29 | I₃₉-b | 0.065 |
| Cf1109 | 0.13 | I₁₉ | 0.008 |
| I₁₄ | 5.28 | **GS-7340** | 0.071 |
| I₁₅ | 0.12 | | |

### Example 41: Cytotoxicity evaluation in vitro

Hep G 2.2.15 cells were inoculated in a 96-well plate (with cell number being 5.5 X 10⁴) to continually cultivate for 3 days, new culture solutions containing different concentrations of drugs were added, and 3 wells were provided in parallel; at day 3 post-dosing, MTT was added until 7.5 mg/ml, and continued to cultivate for 2 hours, the supernate was discarded, isopropanol containing 10% Tween X-100 was added, 120µl/well, and 0.4 µl/well was further added. The absorbance at 540 nm was determined using the enzyme-link meter, and the 50% CC₅₀ value was calculated. The results are shown in Table 2.

**Table 2 Cytotoxicity evaluation results**

| Compound | CC₅₀ (µM) | Compound | CC₅₀ (µM) |
|---|---|---|---|
| i-1 | >200 | I₁₉ | 145.5 |
| i-2 | 152.4 | I₂₀ | 178.9 |
| Cf1109 | 82.0 | I₂₁ | >200 |
| **GS-7340** | 94.2 | I₂₂ | >200 |
| PSI-7977 | 178 | I₂₃ | >200 |
| PSI-353661 | 149 | I₂₄ | >200 |
| i-3 | 177 | I₂₅ | >200 |
| i-4 | 49.7 | I₂₆ | >200 |
| i-5 | 193 | I₂₇ | >200 |
| I₁ | >200 | I₂₈ | >200 |
| I₂ | >200 | I₂₉ | >200 |
| I₃ | >200 | I₃₀ | >200 |
| I₄ | >200 | I₃₁ | >200 |
| I₅ | >200 | I₃₂ | >200 |
| I₆ | >200 | I₃₃ | >200 |
| I₇ | >200 | I₃₄ | >200 |
| I₈ | >200 | I₃₅ | >200 |
| | | I₃₆ | >200 |
| | | I₃₇ | 193 |
| I₁₁ | >200 | I₃₈-a | 139 |
| I₁₂ | >200 | I₃₈-b | >200 |
| I₁₃ | >200 | I₃₉ | 182 |
| I₁₄ | >200 | I₄₀ | >200 |
| I₁₅ | >200 | I₄₁ | >200 |
| I₁₆ | >200 | | |
| I₁₇ | >200 | | |

### Example 42: The evaluation of liver targeting property

Active drug (free phosphonic acid or free phosphoric acid) concentration/content in blood and liver tissues of the title compound at the different time points after intragastric administration for mouse was measured using High Performance Liquid Chromatography-Tandem Mass Spectrometry (HPLC-MS/MS), to calculate the ratio of the drug concentration in liver/bood, thereby comparing the liver targeting property.

Instrument: Finnigan Co. USA, TSQ Quantum-type liquid chromatography-mass spectrometry (LC/MS/MS), consisting of Finnigan Surveyor LC pump, Surveyor AS automatic sampler, electrospray ionization source (ESI) and triple tandem mass spectrometer. The control software is X calibur 1.4, Lcquan 2.0 data processing system is used for mass spectrometry data analysis. The chromatography column is Discovery ODS column (250 mm × 4.6 mm, 5 µm), C18 guard column (4 mm×3.0 mm), the mobile phase is methanol-water-formic acid (10-30:90-70:0.5, V/V/V), the flow rate is 0.7 ml/min; the sample amount is 20 µL; and the column temperature is room temperature.

### Animal experiment

Balb/C mice, male, were fasted for 16 h, randomly divided into 3 groups, 3/group, and intragastrically administrated with fosfomycin (200 mg/kg) or a equimolar dose of a suspension of the title compound in sodium carboxymethylcellulose, respectively, and blood samples were collected at 1 hour and 6 hours post-dosing, respectively, taking serum by centrifugation, while liver tissues were sampled to prepare homogenate, taking supernate by centrifugation; active drug (free phosphonic acid or free phosphoric acid) concentration/content in blood and liver tissues were measured, to calculate the ratio of the drug concentration in liver/bood. The results are shown in Table 3.

**Table 3 The results of evaluation of liver targeting property**

| compound | Cₗᵢᵥₑᵣ/C_{blood} | | compound | Cₗᵢᵥₑᵣ/C_{blood} | |
|---|---|---|---|---|---|
| | 1h | 6h | | 1h | 6h |
| Cf1109 | 2.67 | 4.15 | I₂₉ | 5.18 | 6.92 |
| **GS-7340** | 6.12 | 8.46 | I₃₁-a | 3.40 | 4.11 |
| PSI-7977 | 3.40 | 4.11 | I₃₁-b | 3.40 | 4.11 |
| PSI-353661 | 2.28 | 4.72 | I₃₄-a | 8.19 | 11.2 |
| i-3 | 2.43 | 7.41 | I₃₄-b | 6.36 | 5.18 |
| i-4 | 4.76 | 3.19 | I₃₆ | 10.03 | 7.08 |
| i-5 | 3.17 | 4.08 | I₃₈-a | 7.55 | 10.54 |
| I₂₁-a | 8.45 | 12.02 | I₃₈-b | 7.46 | 6.86 |
| I₂₁-b | 9.15 | 14.37 | | | |
| I₂₆ | 6.97 | 4.38 | | | |
| I₂₇ | 11.75 | 8.02 | | | |

### Example 43: The evaluation of the effect against the liver damage in mice caused by CCl₄

Kunmin mice (20 g) were grouped randomly (8/group). 0.2 mmol of a compound to be tested was orally administered; after 1 hour post-dosing, a 0.1% peanut oil solution of CCl₄ was subcutaneously injected (10 mL/kg), to prepare a liver damage model; the normal saline was injected as a normal control group. After modeling for 12 hours, 0.2 mmol of the compound to be tested was orally administrated again. After 24 hours from the second administration, blood samples were collected to take serum specimen so as to determine ALT, AST. The results are shown in Table 4.

**Table 4 The effect against the liver damage in mice caused by CCl₄ poisoning**

| Compound | ALT(U/L) | AST(U/L) | Compound | ALT(U/L) | AST(U/L) |
|---|---|---|---|---|---|
| Normal control | 75 | 946 | normal saline | 1562 | 2408 |
| i-1 | 2930 | 2943 | I₁ | 328 | 433 |
| i-2 | 3482 | 3660 | I₂ | 435 | 764 |
| Cf1109 | 2048 | 3351 | I₂₉ | 307 | 561 |
| **GS-7340** | 2483 | 2645 | I₃₁-a | 507 | 446 |
| PSI-7977 | 2324 | 2538 | I₃₁-b | 205 | 439 |
| PSI-353661 | 1669 | 2312 | I₃₄-a | 293 | 763 |
| i-3 | 231 | 446 | I₃₄-b | 289 | 536 |
| i-4 | 102 | 592 | I₃₆ | 490 | 543 |
| i-5 | 551 | 641 | I₃₈-a | 410 | 289 |
| I₂₁-a | 353 | 191 | I₃₈-b | 162 | 387 |
| I₂₁-b | 251 | 152 | | | |
| I₂₆ | 521 | 363 | | | |
| I₂₇ | 663 | 940 | | | |

### Example 44: The evaluation of the effect against the liver damage in mice caused by D-aminogalactose

Kunmin mice (20 g) were grouped randomly (8/group). 0.2 mmol of a compound to be tested was orally administered; after 2 hours post-dosing, D-aminogalactose was intraperitoneally injected at a dose of 750 mg/kg to prepare a liver damage model, and the normal saline was subcutaneously injected as a normal control group. After modeling for 12 hours, 0.2 mmol of the compound to be tested was orally administrated again. After 24 hours from the second administration, blood samples were collected to take serum specimen so as to determine ALT, AST. The results are shown in Table 5.

**Table 5 The effect against the liver damage in mice caused by D-aminogalactose**

| Compound | ALT(U/L) | AST(U/L) | Compound | ALT(U/L) | AST(U/L) |
|---|---|---|---|---|---|
| Normal control | 58 | 193 | Normal saline | 1255 | 1490 |
| i-1 | 1227 | 1456 | I₁ | 679 | 796 |
| i-2 | 1374 | 1812 | I₂ | 514 | 560 |
| Cf1109 | 191 | 140 | I₂₉ | 301 | 510 |
| **GS-7340** | 1064 | 1320 | I₃₁-a | 421 | 253 |
| PSI-7977 | 1079 | 1727 | I₃₁-b | 375 | 751 |
| PSI-353661 | 1558 | 1027 | I₃₄-a | 131 | 282 |
| i-3 | 1150 | 1419 | I₃₄-b | 341 | 194 |
| i-4 | 1451 | 1356 | I₃₆ | 187 | 113 |
| i-5 | 1512 | 1646 | I₃₈-a | 177 | 275 |
| I₂₁-a | 379 | 169 | I₃₈-b | 217 | 493 |
| I₂₁-b | 514 | 268 | I₄₂ | 1491 | 346 |
| I₂₆ | 212 | 586 | I₄₃ | 256 | 517 |
| I₂₇ | 713 | 522 | | | |

### Example 45: The evaluation of anti-HBV effect in vivo

The sheldrakes infected by vertical transmission and being positive in DHBV DNA detection were grouped randomly, 5/group. Water and different doses of the compound to be tested were intragastrically administrated, respectively, once a day, for 14 days. Venous blood samples were collected pre-dosing, at day 14 of dosing and at day 7 after drug withdrawal, the serum DHBV DNA content was measured using external standard TaqMan real-time fluorescence PCR method, and the inhibition ratio was calculated, as compared to the solvent group. The results are shown in Table 6:

**Table 6 The effect against the liver damage in mice caused by D-aminogalactose**

| Compound | Dose mg/kg | Inhibition ratio (%) | | Compound | Dose mg/kg | Inhibition ratio (%) | |
|---|---|---|---|---|---|---|---|
| | | Day 14 of dosing | day 7 after drug withdrawal | | | Day 14 of dosing | day 7 after drug withdrawal |
| Lamivudine | 20 | 76.1 | 2.35 | I₂₆ | 25 | 93.5 | 11.5 |
| Acyclovir | 20 | 12.5 | 2.64 | I₂₇ | 10 | 80.6 | 10.5 |
| Cf1109 | 20 | 68.8 | 5.38 | I₂₉ | 10 | 90.8 | 17.1 |
| **GS-7340** | 50 | 85.2 | 23.1 | I₃₆ | 20 | 92.0 | 10.8 |
| i-3 | 20 | 56.6 | 14.6 | I₃₇ | 10 | 75.2 | 9.4 |
| i-4 | 1.0 | 49.0 | 7.30 | I₃₈-a | 0.5 | 76.2 | 31.4 |
| i-5 | 1.0 | 88.9 | 15.70 | I₃₈-b | 0.5 | 96.4 | 63.4 |
| I₂₁-a | 25 | 70.8 | 25.0 | I₄₁ | 10 | 72.5 | 15.7 |
| I₂₁-b | 25 | 82.3 | 39.2 | | | | |

## Claims

1. A phosphonic acid derivative, a pharmaceutically acceptable salt or a solvate thereof ,which is selected from the following structures:

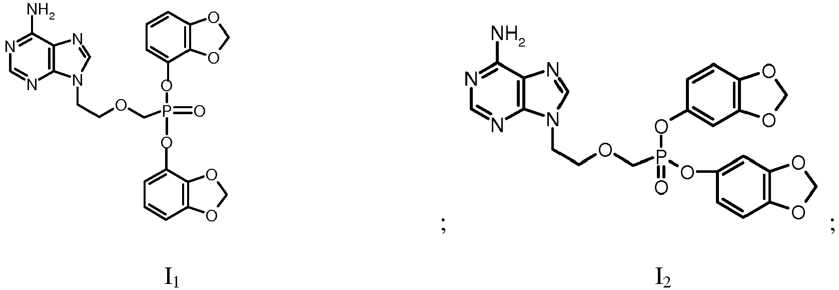

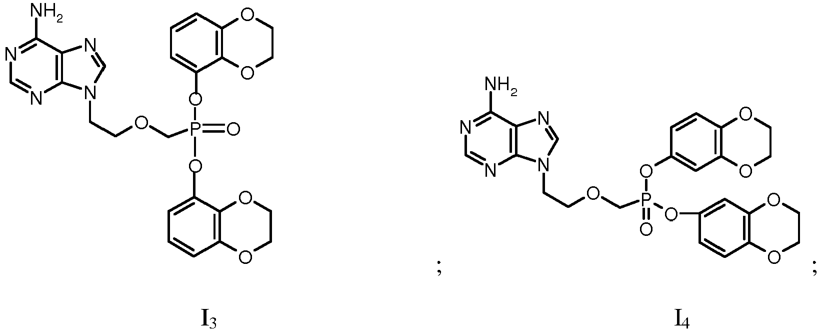

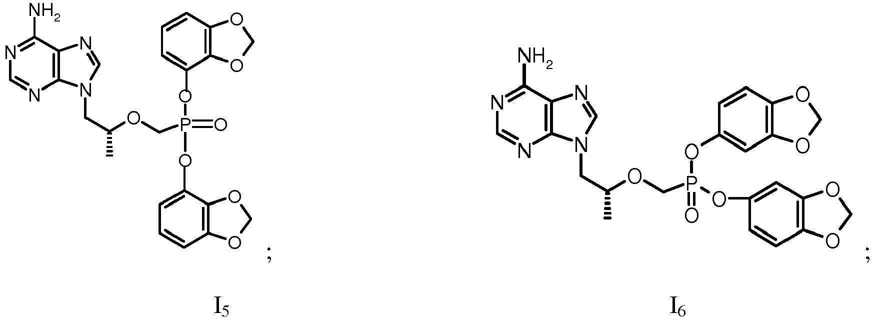

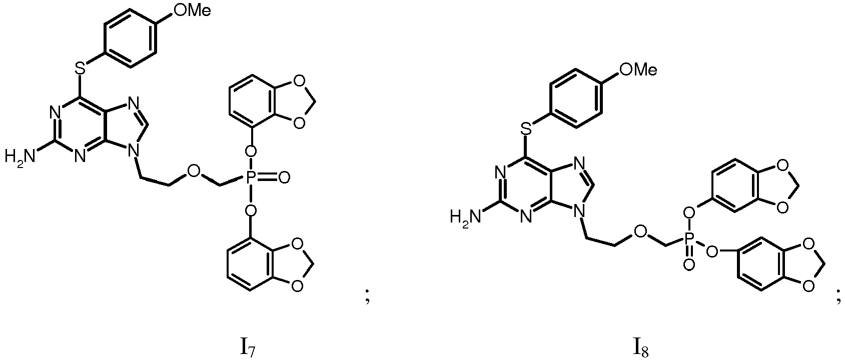

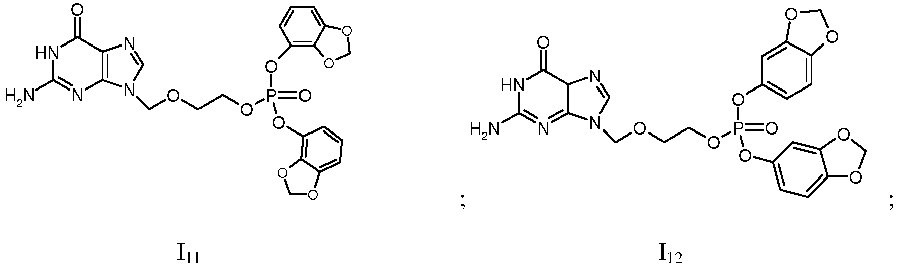

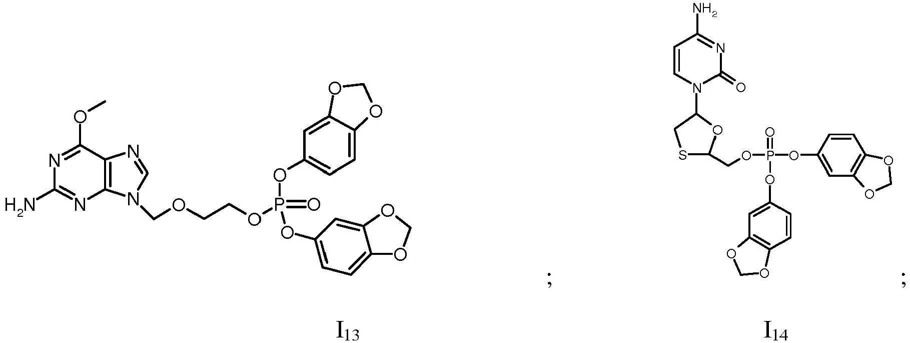

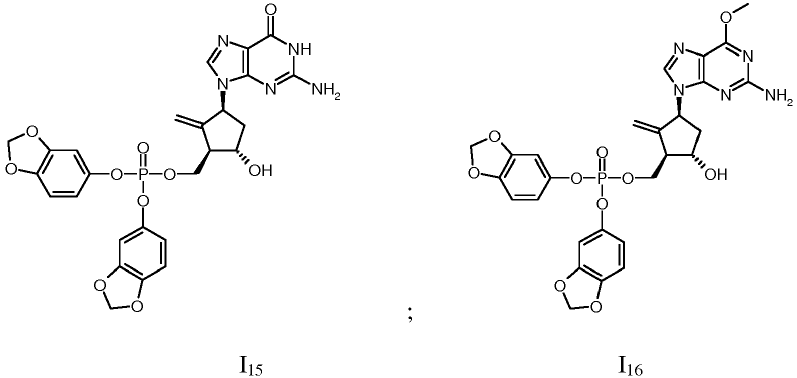

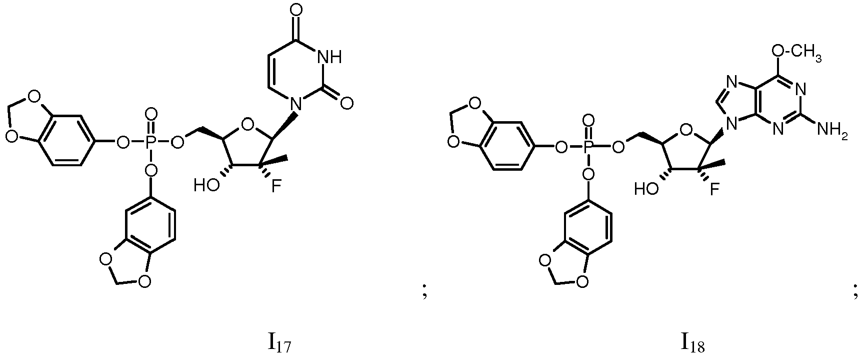

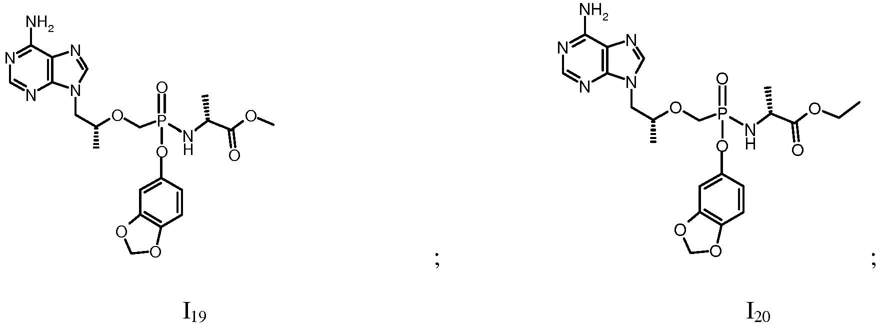

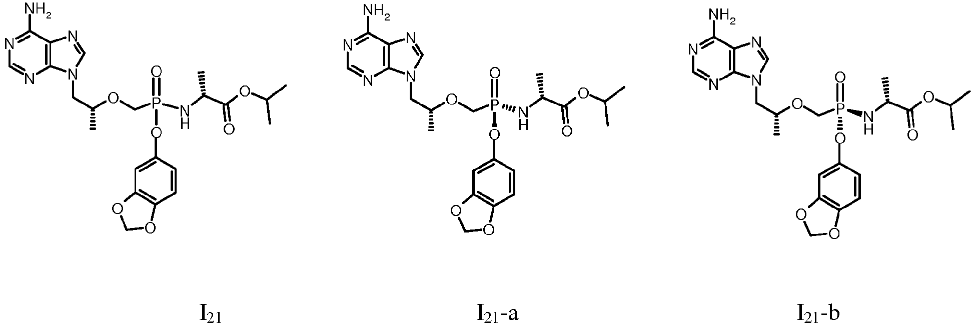

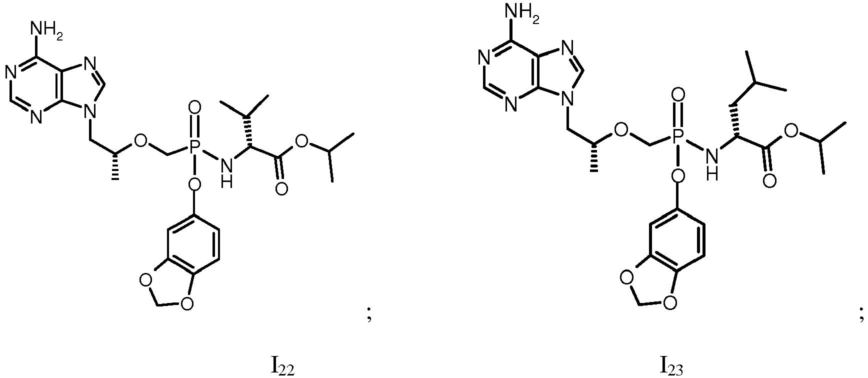

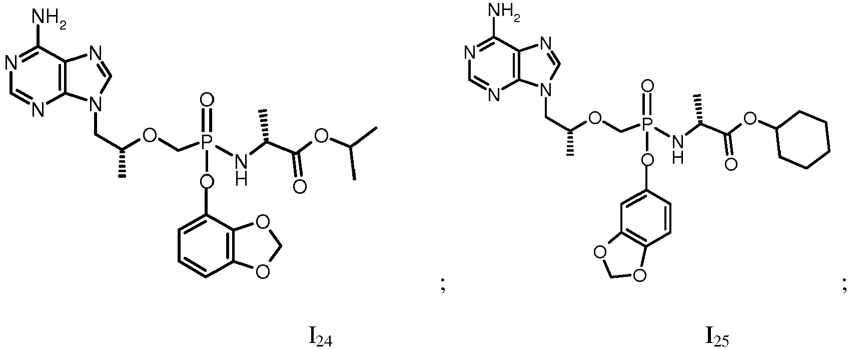

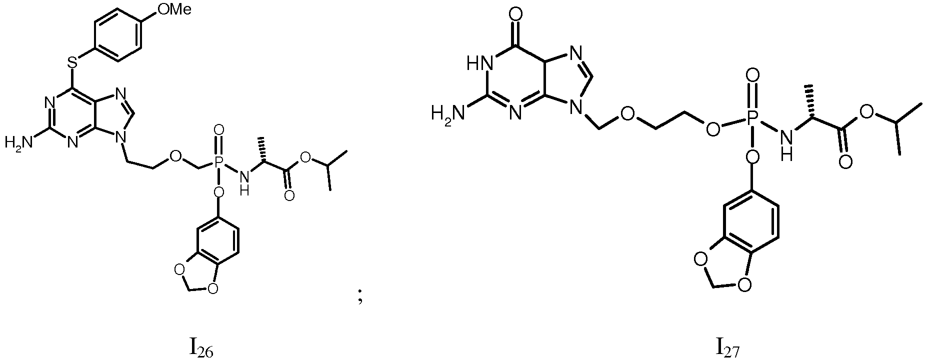

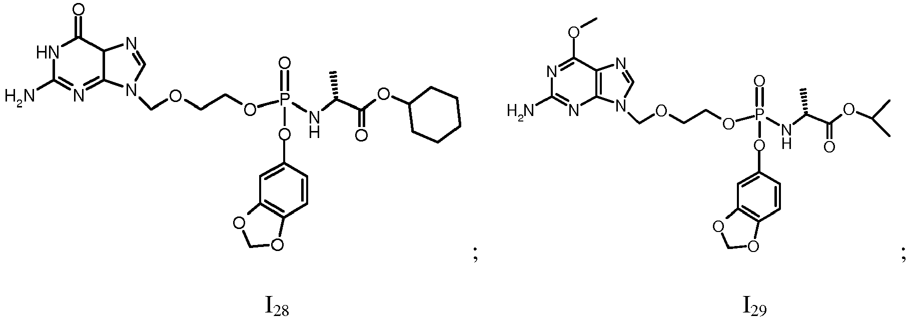

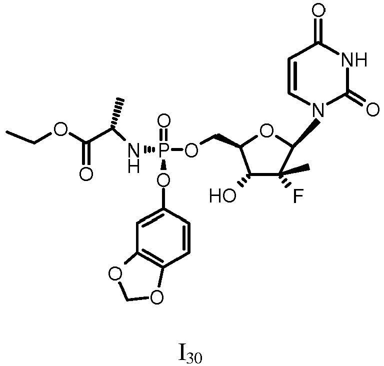

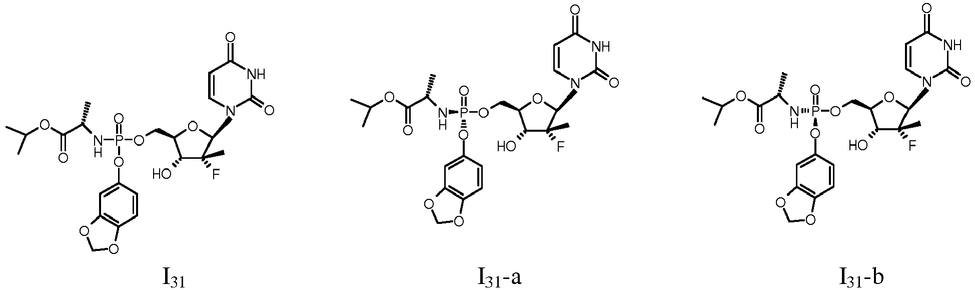

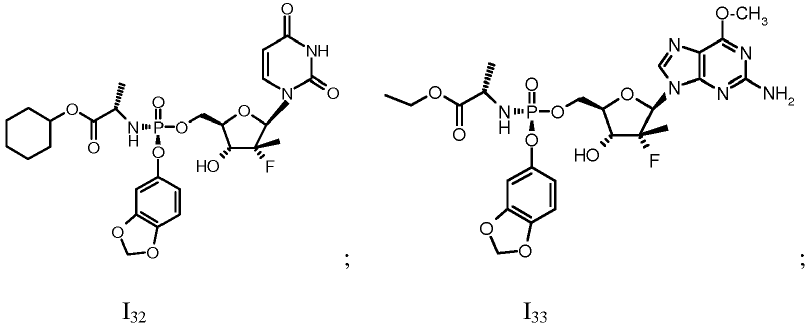

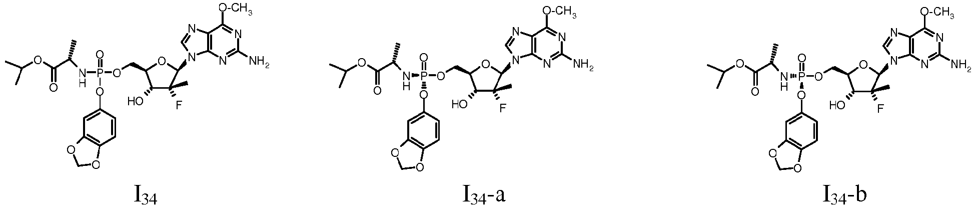

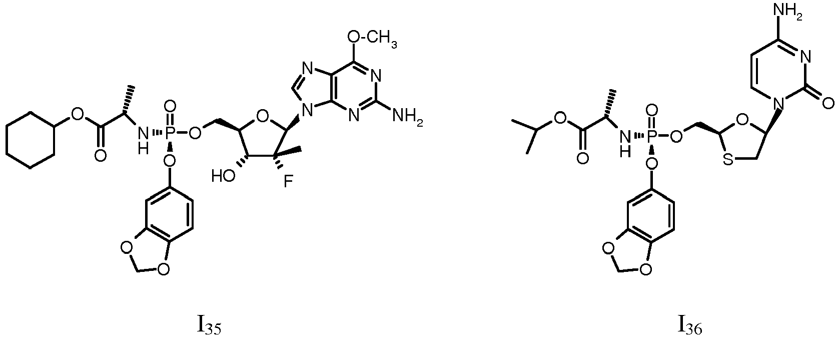

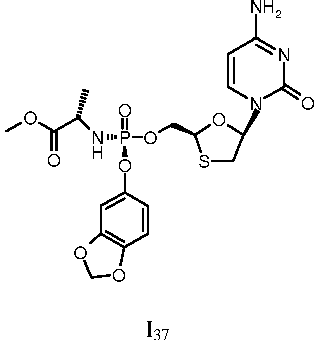

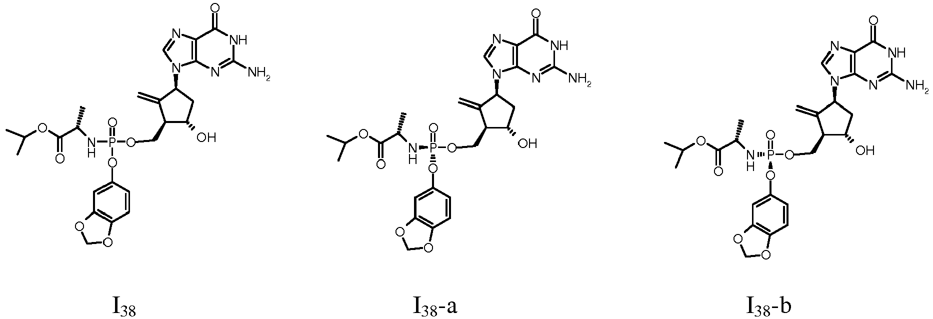

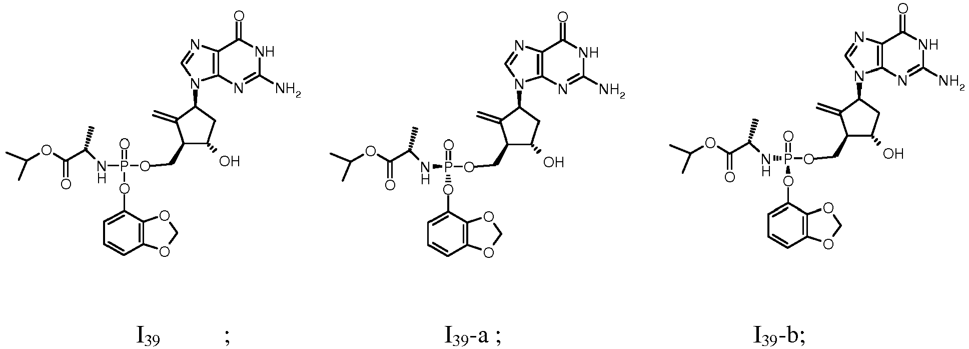

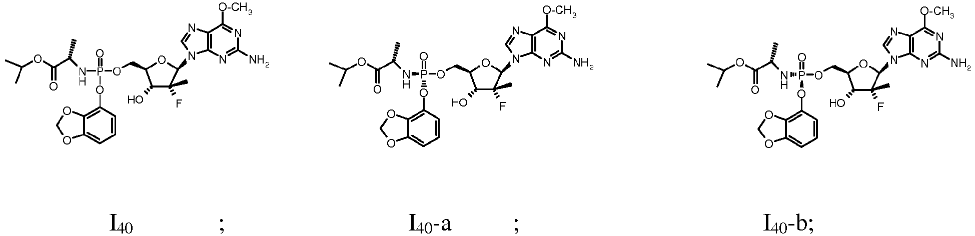

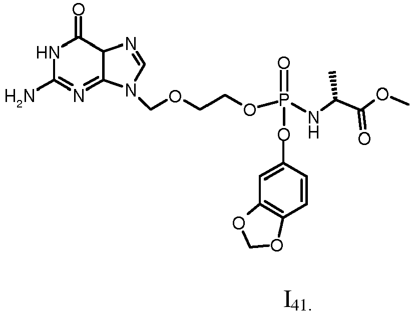

2. Pharmaceutical composition comprising the compound or a pharmaceutically acceptable salt or a solvate thereof according to claim 1 as an active ingredient and one or more pharmaceutical carriers or excipients.

3. Use of the phosphonic acid derivative,a pharmaceutically acceptable salt or a solvate thereof according to claim 1 in the preparation of a medicament for treating hepatitis.

4. Use of the phosphoric acid derivative, a pharmaceutically acceptable salt or a solvate thereof according to claim 1 in the preparation of a medicament for treating viral hepatitis.

## Patentansprüche

1. Phosphonsäurederivat, ein pharmazeutisch annehmbares Salz oder ein Solvat davon, das aus den folgenden Strukturen ausgewählt ist:

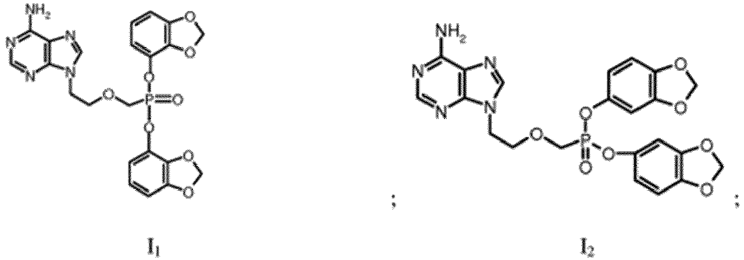

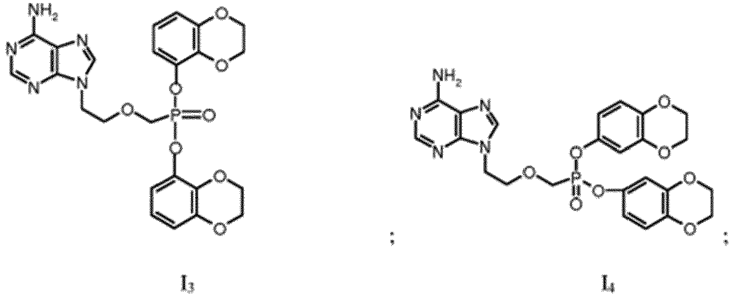

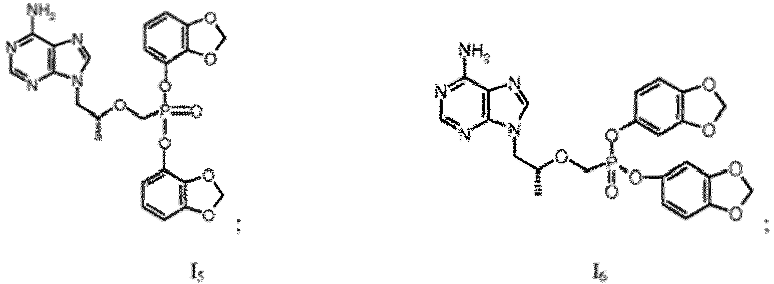

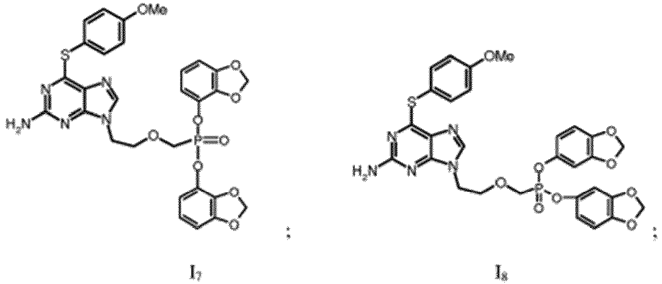

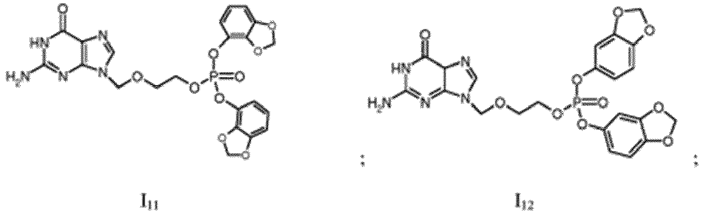

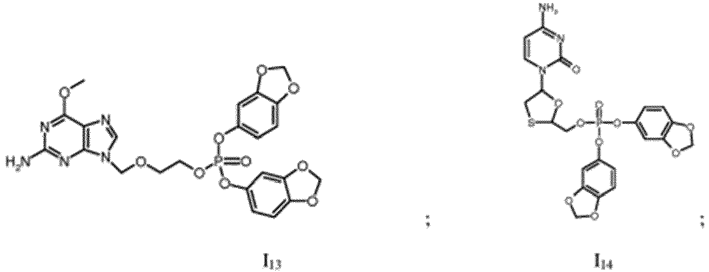

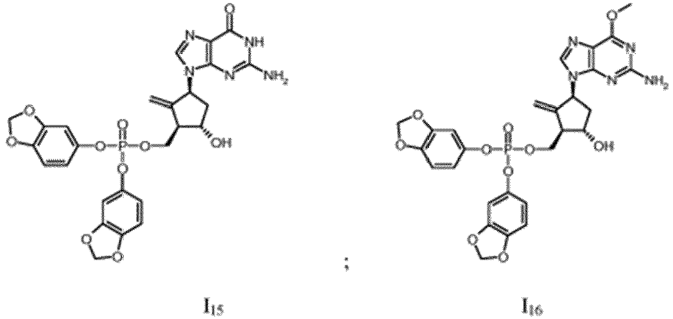

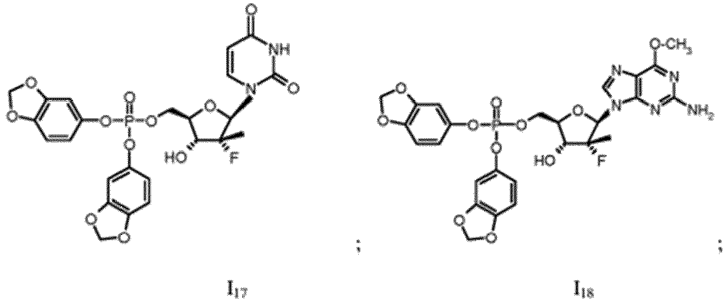

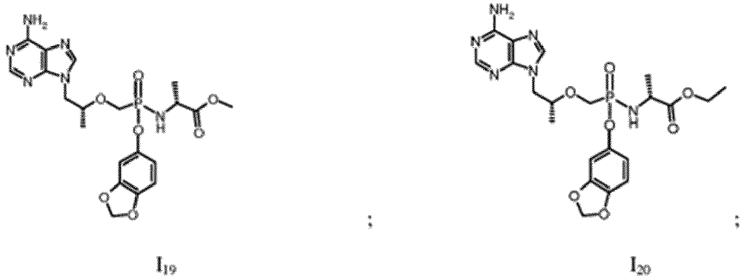

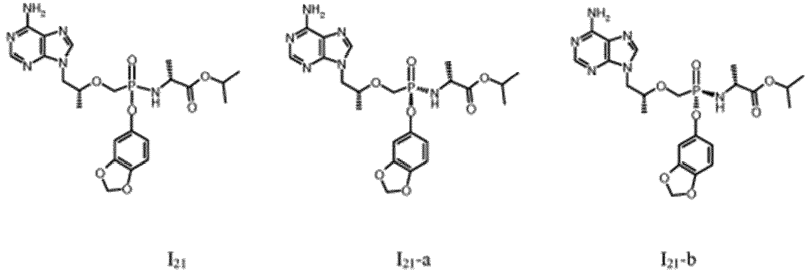

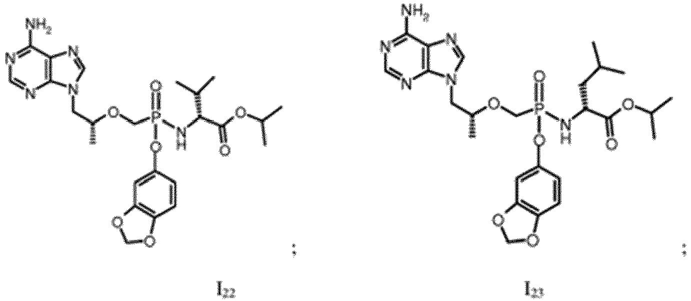

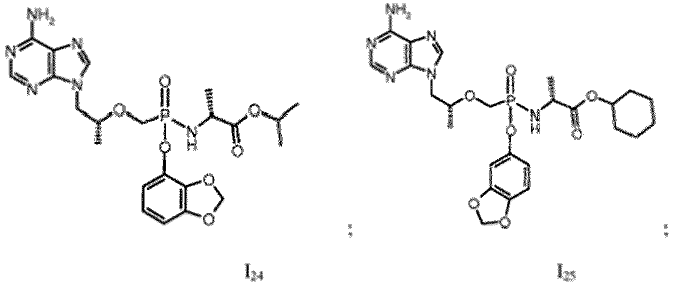

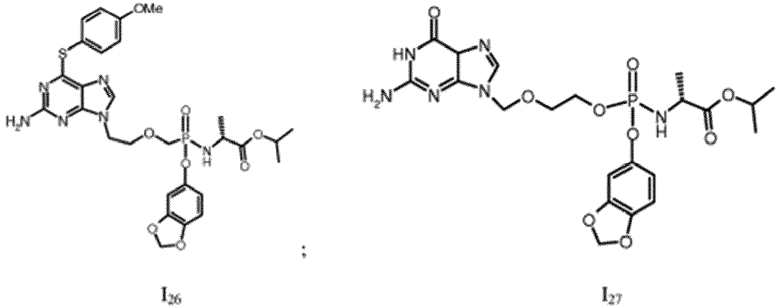

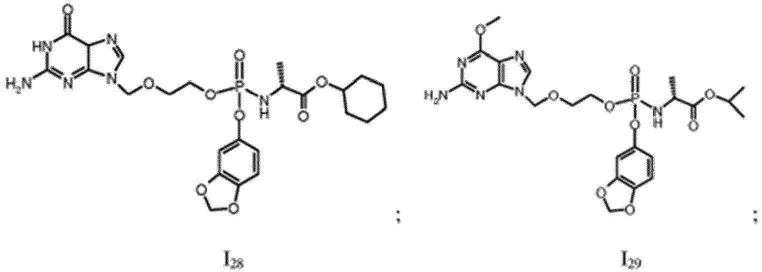

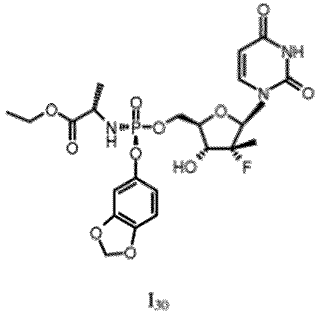

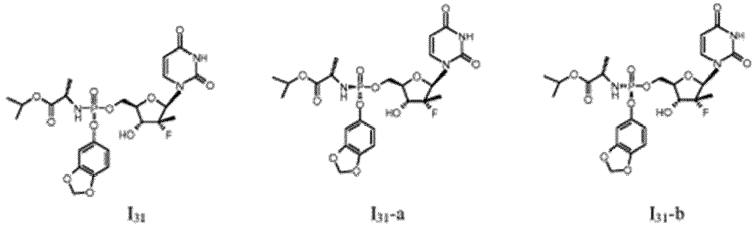

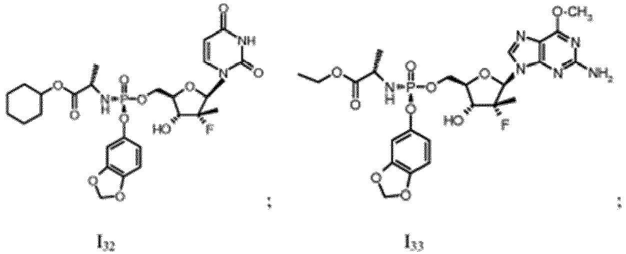

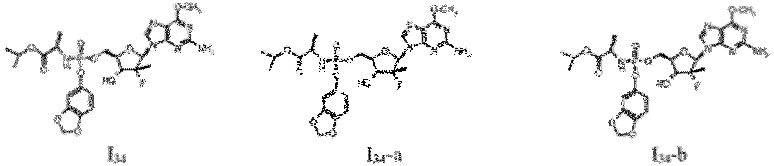

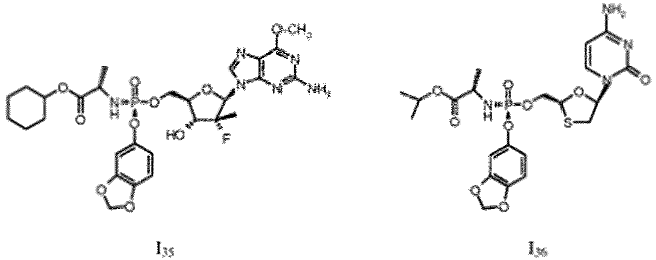

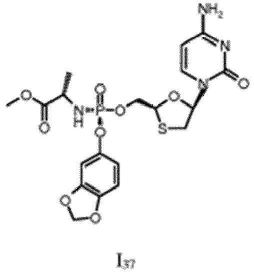

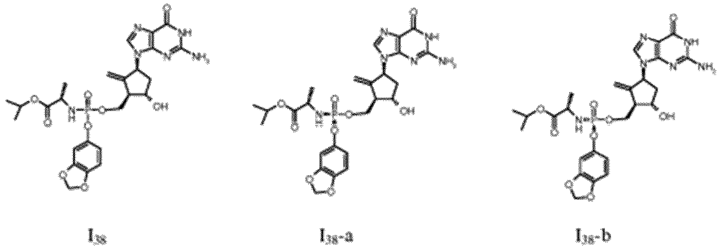

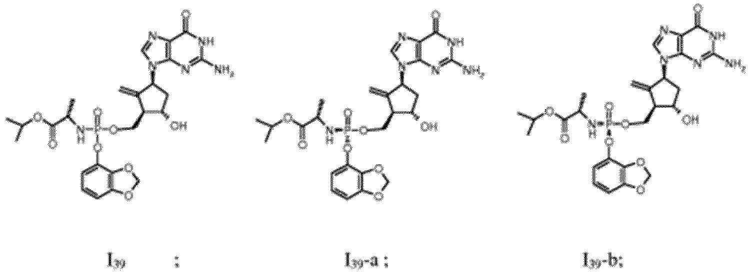

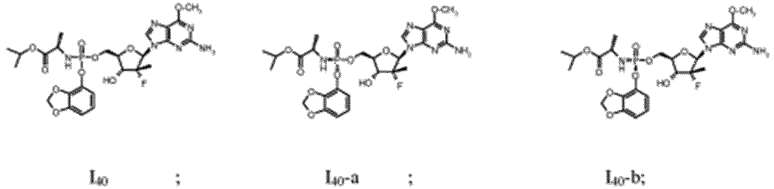

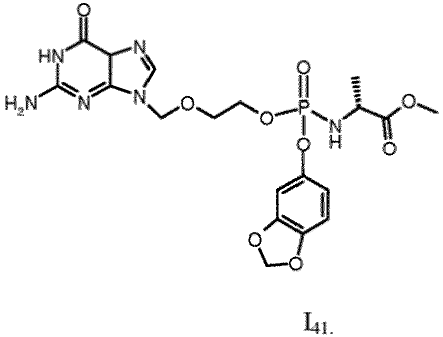

2. Pharmazeutische Zusammensetzung, umfassend die Verbindung oder ein pharmazeutisch annehmbares Salz oder ein Solvat davon nach Anspruch 1 als aktiven Bestandteil und einen oder mehrere pharmazeutische Träger oder Exzipienten.

3. Verwendung des Phosphonsäurederivats, eines pharmazeutisch annehmbaren Salzes oder eines Solvats davon nach Anspruch 1 bei der Herstellung eines Medikaments zum Behandeln von Hepatitis.

4. Verwendung des Phosphonsäurederivats, eines pharmazeutisch annehmbaren Salzes oder eines Solvats davon nach Anspruch 1 bei der Herstellung eines Medikaments zum Behandeln von Virushepatitis.

## Revendications

1. Dérivé d'acide phosphonique, un sel pharmaceutiquement acceptable ou un solvate de celui-ci, qui est choisis parmi les structures suivantes:

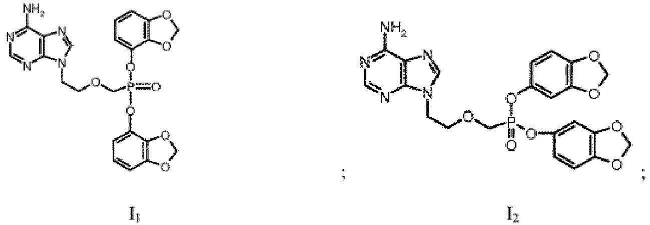

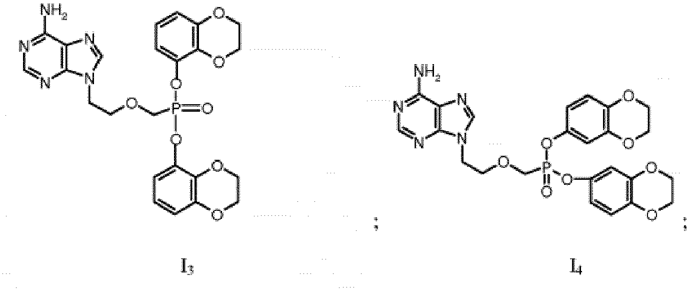

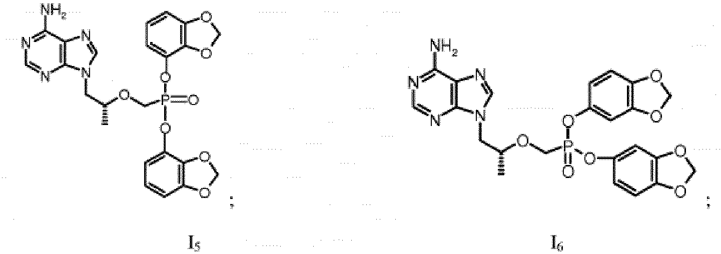

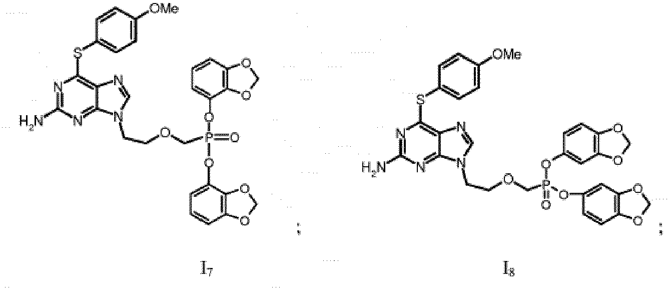

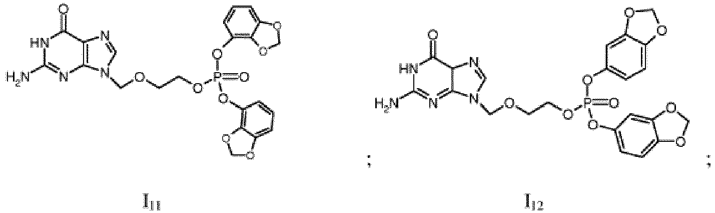

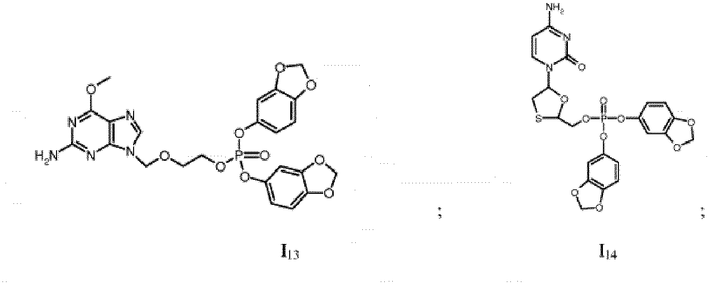

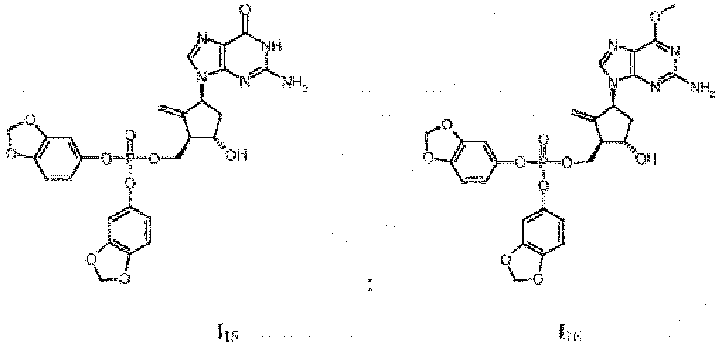

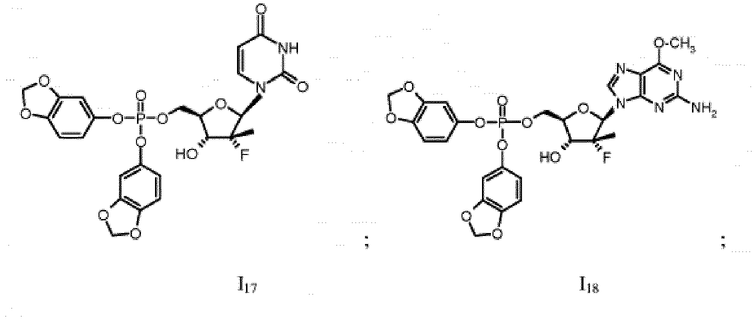

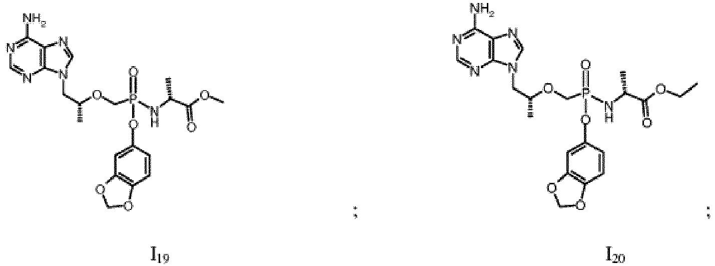

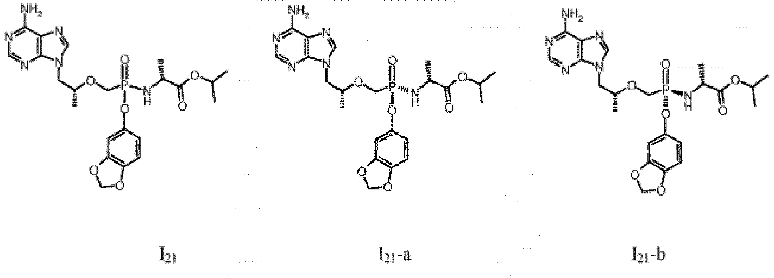

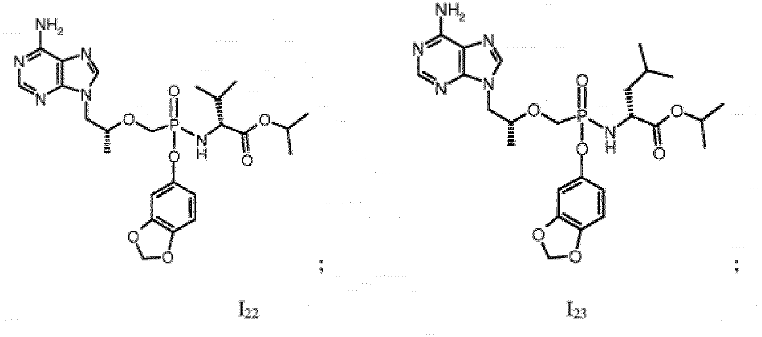

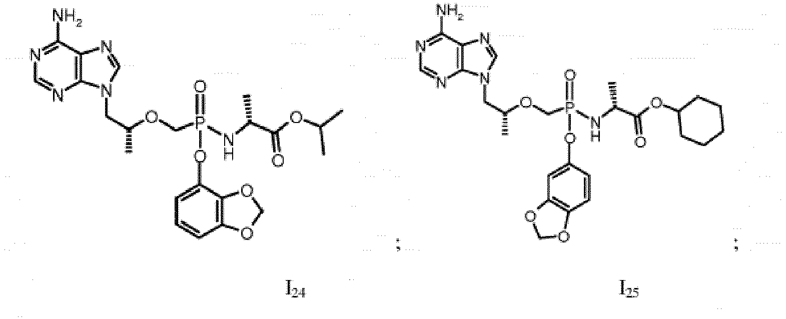

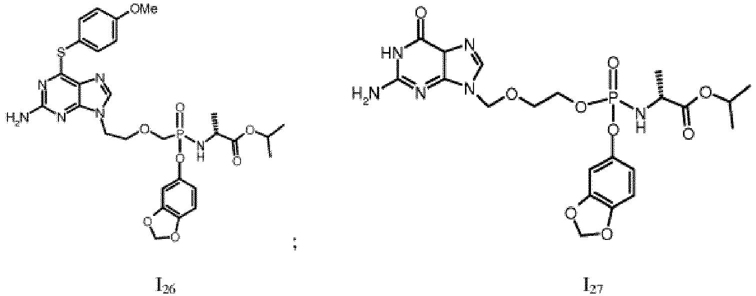

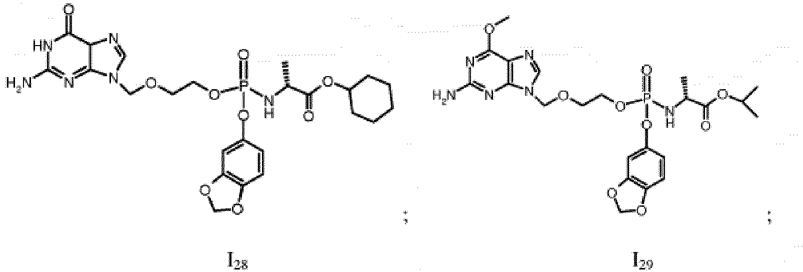

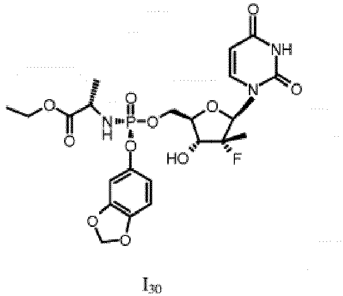

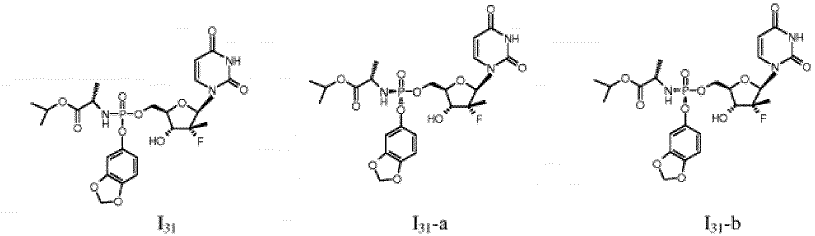

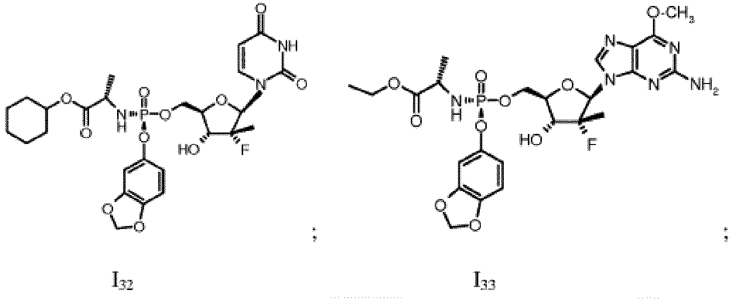

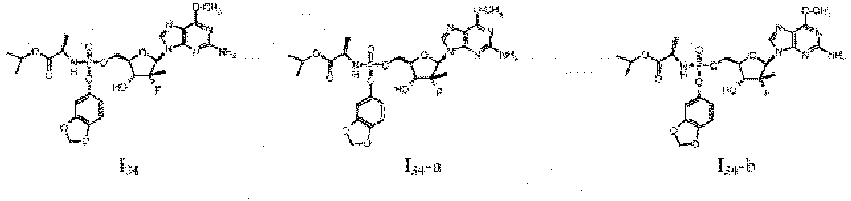

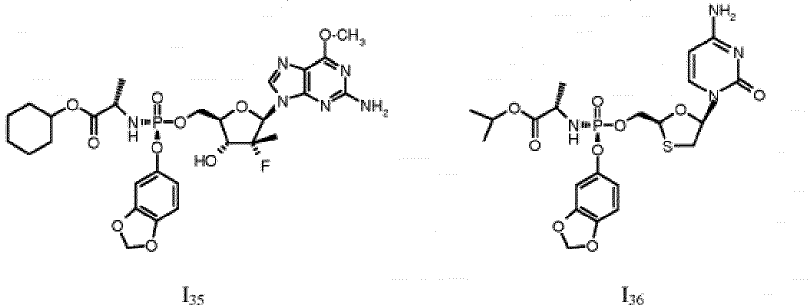

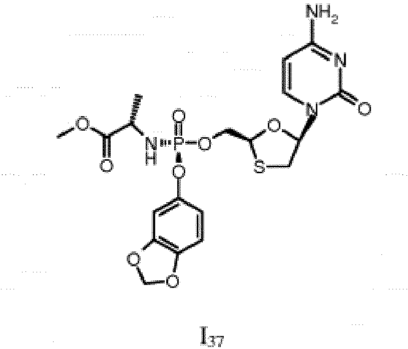

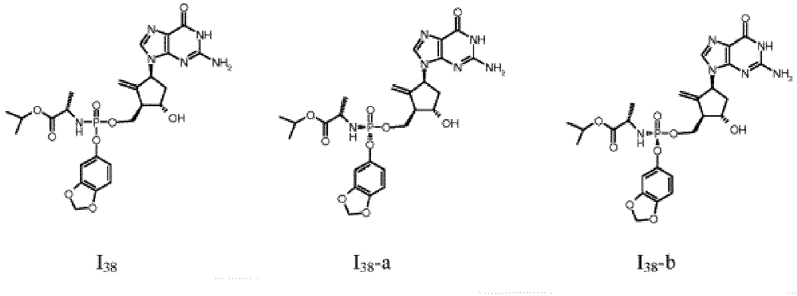

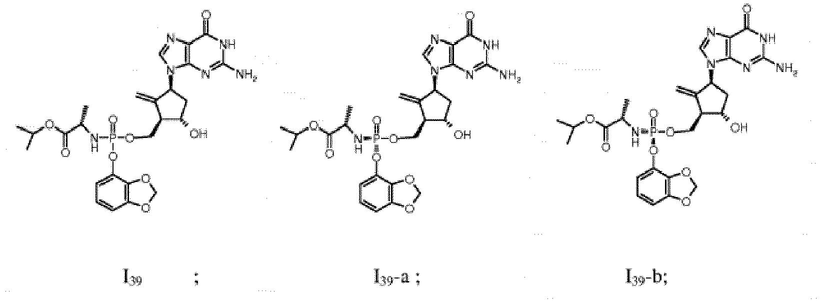

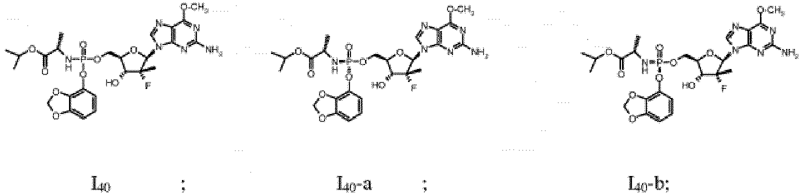

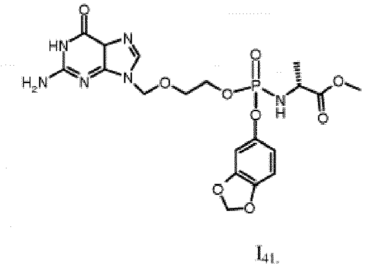

2. Composition pharmaceutique comprenant le composé ou un sel pharmaceutiquement acceptable ou un solvate de celui-ci selon la revendication 1 en tant qu'ingrédient actif et un ou plusieurs véhicules ou excipients pharmaceutiques.

3. Utilisation du dérivé d'acide phosphonique, d'un sel pharmaceutiquement acceptable ou d'un solvate de celui-ci selon la revendication 1 dans la préparation d'un médicament pour le traitement de l'hépatite.

4. Utilisation du dérivé d'acide phosphonique, d'un sel pharmaceutiquement acceptable ou d'un solvate de celui-ci selon la revendication 1 dans la préparation d'un médicament pour le traitement de l'hépatite virale.
